# EUROPEAN PATENT SPECIFICATION

(11) **EP 3 244 932 B1**
(45) Date of publication and mention of the grant of the patent: **05.03.2025**
(21) Application number: 16737885.0
(22) Date of filing: 14.01.2016
(51) Int. Cl.: C12N 15/113, C12N 15/90, A61K 38/16, C12N 9/22, A61P 31/22, A61P 43/00

(54) **RNA GUIDED ERADICATION OF HERPES SIMPLEX TYPE I AND OTHER RELATED HERPESVIRUSES**
RNA-GEFÜHRTE AUSROTTUNG DES HERPES SIMPLEX VIRUS TYP I UND ANDERER VERWANDTER HERPESVIREN
ÉRADICATION DE L'HERPÈS SIMPLEX DE TYPE I ET D'AUTRES VIRUS DE L'HERPÈS ASSOCIÉS GUIDÉE PAR ARN

(30) Priority: 14.01.2015 US 201562103354 P; 07.10.2015 US 201562238288 P
(43) Date of publication of application: 22.11.2017
(73) Proprietor: Temple University Of The Commonwealth System Of Higher Education, Philadelphia, PA 19122 (US)
(72) Inventor: ROEHM, Pamela, C., Jenkintown, PA 19046 (US); KHALILI, Kamel, Bala Cynwyd, PA 19004 (US); SHEKARABI, Sayed, Masoud, Lansdale, PA 19446 (US); WOLLEBO, Hassen, Philadelphia, PA 19134 (US)
(74) Representative: Inspicos P/S
(86) International application number: PCT/US2016/013423
(87) International publication number: WO 2016/115355

(56) References cited:
- WO-A1-2015/153789
- WO-A1-2015/153791
- WO-A1-2017/075475
- WO-A2-2014/191521
- WO-A2-2015/126927
- WO-A2-2015/153889
- US-A1- 2009 068 215
- US-A1- 2014 068 797
- YANWEI BI ET AL: "High-Efficiency Targeted Editing of Large Viral Genomes by RNA-Guided Nucleases", PLOS PATHOGENS, vol. 10, no. 5, 1 May 2014 (2014-05-01), pages e1004090, XP055198358, DOI: 10.1371/journal.ppat.1004090
- ET AL. CONG: "Addgene: pX330-U6-Chimeric _ BB-CBh-hSpCas9", ADDGENE'S BLOG, 3 January 2013 (2013-01-03), pages 1 - 5, XP055377318, Retrieved from the Internet <URL:http://www.addgene.org/42230/> [retrieved on 20170531]
- TADAHIRO SUENAGA ET AL: "Engineering large viral DNA genomes using the CRISPR-Cas9 system", MICROBIOLOGY AND IMMUNOLOGY, vol. 58, no. 9, 1 September 2014 (2014-09-01), pages 513 - 522, XP055205700, ISSN: 0385-5600, DOI: 10.1111/1348-0421.12180
- J. WANG ET AL: "RNA-guided endonuclease provides a therapeutic strategy to cure latent herpesviridae infection", PROCEEDINGS OF THE NATIONAL ACADEMY OF SCIENCES, vol. 111, no. 36, 25 August 2014 (2014-08-25), pages 13157 - 13162, XP055206047, ISSN: 0027-8424, DOI: 10.1073/pnas.1410785111
- STEPHANIE GROSSE ET AL: "Meganuclease-mediated Inhibition of HSV1 Infection in Cultured Cells", MOLECULAR THERAPY, vol. 19, no. 4, 1 April 2011 (2011-04-01), pages 694 - 702, XP055071509, ISSN: 1525-0016, DOI: 10.1038/mt.2010.302
- WANG ET AL.: "RNA-guided endonuclease provides a therapeutic strategy to cure latent herpesviridae infection.", PROC NATL ACAD SCI USA., vol. 111, no. 36, 2014, pages 13157 - 13162, XP055206047
- FONFARA ET AL.: "Phylogeny of Cas9 determines functional exchangeability of dual-RNA and Cas9 among orthologous type II CRISPR-Cas systems.", NUCLEIC ACIDS RES., vol. 42, no. 4, 2014, pages 2577 - 90, XP055399937
- JINEK ET AL.: "RNA-programmed genome editing in human cells.", ELIFE., vol. 2, 2013, pages e00471
- DATABASE UniProtKB_Q99 ZW 2 [o] 1 June 2001 (2001-06-01), "CRISPR-associated endonuclease Cas9/Csn1", XP055467792, Database accession no. Q99 ZW 2
- SLAYMAKER ET AL.: "Rationally engineered Cas9 nucleases with improved specificity.", SCIENCE, vol. 351, no. 6268, January 2016 (2016-01-01), pages 84 - 8, XP002757561

## Description

### BACKGROUND OF THE INVENTION

Herpes simplex virus type 1 (HSV-1) is a human neurotropic virus that infects the majority of the human population worldwide (Whitley and Roizman, 2001, Lancet 357: 1513-1518; Xu et al, 2006, JAMA, 296: 964-973). The HSV-1 genome is present in 85-90% of trigeminal ganglia of individuals at autopsy (Baringer, 2000, Infectious diseases of the nervous system, Butterworth-Heinemann, Oxford: 139-164), and its seroprevalence is 90% in normal asymptomatic individuals (Kennedy and Chaudhuri, 2002, J Neurol Neurosurg Psychiatry, 73: 237-238). In most cases, after initial infection HSV-1 persists in only a latent stage, without causing obvious symptoms. However, in some cases the primary infection causes fever, dysphagia, odynophagia, and painful blisters on the lips and tongue (Kennedy and Steiner, 2013, J Neurovirol, 19: 346-350; Whitley and Roizman, 2001, Lancet 357: 1513-1518), and in neonates, primary HSV-1 infection can lead to significant morbidity and mortality (Westerberg et al, 2008, Int J Pediatr Otorhinolaryngol, 72: 931-937; Whitley and Roizman, 2001, Lancet 357: 1513-1518). Replication of HSV-1 in the nervous system causes encephalitis with symptoms ranging from fever, focal deficits, aphasia and seizures, resulting from the infection and anti-viral inflammatory response within the frontal lobe and the temporal lobe (Gilden et al, 2007, Nature Clin Practice 3, 82-94). The site of HSV-1 latency appears mainly restricted to cranial nerve ganglia, although in some cases the viral genome has been detected in thoracic ganglia and brain (Fraser et al., 1981, Proc Natl Acad Sci USA, 78: 6461-6465; Mahalingam et al, 1992, Ann Neurol, 31: 444-448). Current treatments for primary HSV-1 infection and reactivation of diseases are non-selective, do not prevent establishment of latent infection or viral reactivation, and have adverse side effects, pointing to a strong need for improved and specific therapeutic strategies.

The mechanisms of HSV latency initiation and persistence are not fully understood, but evidence points to a role of viral RNA elements (latency-associated transcripts; LATS), which are abundantly expressed during latency (Izumi et al, 1989, Microb Pathol, 7: 121-134; Kang et al, 2003, Virol, 312: 233-244; Perng et al, 2000, Science 287: 1500-1503; Stevens et al, 1987, Science, 235: 1056-1059). Environmental factors including UV light stimulation, hyperthermia, social stress and a host of pharmacological agents can trigger reactivation of the latent HSV-1 genome, productive viral replication in neurons, and disease progression. The most common and easily identifiable clinical sign of HSV-1 reactivation is cold sores (herpes labialis), but other conditions including recurrent genital herpes have been linked to HSV reactivation (Xu et al, 2006, JAMA, 296: 964-973).

Pharmacologic treatment with nucleoside analogues is the mainstay of therapy for HSV1 primary infection and viral reactivation events. The nucleoside analogues with the best therapeutic index target the viral thymidine kinase (TK), including acyclovir and its derivatives. These agents are monophosphorylated by TK, allowing formation of ACV bi- and triphosphates by cellular enzymatic machinery and ultimately resulting in HSV1 DNA chain termination. While these drugs can effectively limit damage resulting from spread of HSV1 infection to other cells, they have no effect on the establishment of latent HSV1 reactivation or on future HSV1 reactivation events. Patients, particularly those with underlying immunodeficiency, can develop HSV1 strains that are resistant to TK inhibitors, and subsequently require treatment with DNA inhibitors that are less HSV1-specific. TK inhibitors are also associated with renal toxicity. Other anti-herpetic agents target the viral DNA polymerase UL30, such as foscarnet and cidofovir. These medications can help control the symptoms of primary HSV1 infection and reactivation, accelerate the process of healing of lesions, and decrease pain and duration of viral shedding. Unfortunately, use of these medications does not eradicate latent HSV1 infection. Development of HSV1 strains that are resistant to anti-herpetic medications occurs, particularly in patients with immunodeficiencies. Resistant strains commonly contain TK mutations; however, UL30 mutations also occur. HSV1 TK inhibitors and UL30 active agents are not effective against HSV1 with UL30 mutations.

Attempts to develop vaccines against HSV1 have met with minimal success (Belshe et al, 2012, N Engl J Med 366: 34-43; Whitley and Roizman, 2001, Lancet 357: 1513-1518). At present there is no FDA approved vaccination for HSV1. Initial trials of vaccination for HSV1 and HSV2 have demonstrated that for these viruses vaccination will likely be most effective in previously uninfected individuals and will likely have minimal efficacy for control of multiple reactivation events in previously infected patients.

Given the limitations of current therapy, there is a need in the art for compositions and methods for the treatment and prevention of both lytic and latent HSV1 infection.

### SUMMARY OF THE INVENTION

In one aspect, the present invention provides a composition for use in treating or preventing a herpesvirus infection. The composition comprises
(a) an isolated nucleic acid encoding a CRISPR-associated (Cas) peptide; and
(b) one or more isolated nucleic acids, wherein the one or more isolated nucleic acids encode multiple guide nucleic acids, wherein each guide nucleic acid comprises a nucleotide sequence complementary to a different target sequence in the herpesvirus genome, and wherein a guide nucleic acid of the multiple guide nucleic acids comprises a target sequence complementary to a sequence within the ICPO domain of the herpesvirus genome and wherein a second guide nucleic acid of the multiple guide nucleic acids comprises a target sequence complementary to a sequence within the ICP4 or ICP27 domain of the herpesvirus genome.

In one embodiment, the Cas peptide is Cas9 or a variant thereof. In one embodiment, the Cas9 variant comprises one or more point mutations, relative to wildtype *Streptococcus pyogenes* Cas9 (spCas9), selected from the group consisting of: R780A, K810A, K848A, K855A, H982A, K1003A, R1060A, D1135E, N497A, R661A, Q695A, Q926A, L169A, Y450A, M495A, M694A, and M698A. In one embodiment, the Cas peptide is Cpf1 or a variant thereof.

In one embodiment, the isolated nucleic acid encoding the Cas peptide is optimized for expression in a human cell.

In one embodiment, the guide nucleic acid is RNA. In one embodiment, the guide nucleic acid comprises crRNA and tracrRNA.

In one embodiment, the herpesvirus is selected from the group consisting of herpes simplex type I (HSV1), herpes simplex virus 2 (HSV2), human herpresvirus-3 (HHV-3; varicella zoster virus (VZV), human herpesvirus-4 (HHV-4; Epstein-Barr virus (EBV)), human herpesvirus-5 (HHV-5; Cytomegalovirus (CMV)), human herpesvirus-6 (HHV-6; roseolovirus), human herpes virus-7 (HHV-7), and human herpesvirus-8 (HHV-8; Karposi's sarcoma-associated herpesvirus (KSHV)).

In one embodiment, the target sequence is selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8.

In one aspect, the present invention provides a pharmaceutical composition comprising
(a) an isolated nucleic acid encoding a CRISPR-associated (Cas) peptide; and
(b) one or more isolated nucleic acids, wherein the one or more isolated nucleic acids encode multiple guide nucleic acids, wherein each guide nucleic acid comprises a nucleotide sequence complementary to a different target sequence in the herpesvirus genome, and wherein a guide nucleic acid of the multiple guide nucleic acids comprises a target sequence complementary to a sequence within the ICPO domain of the herpesvirus genome and wherein a second guide nucleic acid of the multiple guide nucleic acids comprises a second target sequence complementary to a sequence within the ICP4 or ICP27 domain of the herpesvirus genome.

In one aspect, the present invention comprises an expression vector encoding a CRISPR-associated (Cas) peptide and one or more isolated nucleic acids, wherein the one or more isolated nucleic acids encode multiple guide nucleic acids, wherein each guide nucleic acid comprises a nucleotide sequence complementary to a different target sequence in the herpesvirus genome, and wherein a guide nucleic acid of the multiple guide nucleic acids comprises a target sequence complementary to a sequence within the ICPO domain of the herpesvirus genome and wherein a second guide nucleic acid of the multiple guide nucleic acids comprises a second target sequence complementary to a sequence within the ICP4 or ICP27 domain of the herpesvirus genome.

The invention enables a method of treating or preventing a herpesvirus infection or herpesvirus-associated disorder in a subject. The method comprises contacting a cell of the subject with a therapeutically effective amount of a composition defined above.

In one embodiment, the herpesvirus-associated disorder treated is selected from the group consisting of labial herpes, genital herpes, chickenpox, shingles, primary herpes infection with a human alpha-herpesvirus, Bell's palsy, vestibular neuritis, and herpetic neuralgia.

### BRIEF DESCRIPTION OF THE DRAWINGS

The following detailed description of preferred embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities of the embodiments shown in the drawings.

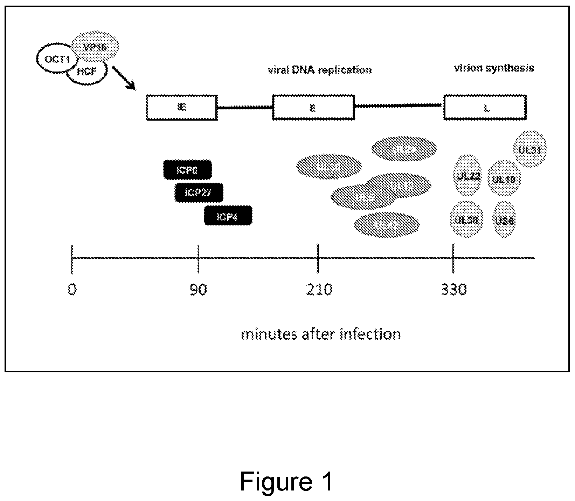

Figure 1 is a graphic representation of the time course of protein expression following lytic HSV1 infection. Shown are representative immediately early (IE, black), early (E, grey) and late (L, light grey) proteins. VP16, a late protein packaged in the viral tegument, interacts with host cell proteins Oct1 and HCF to initiate viral protein synthesis.

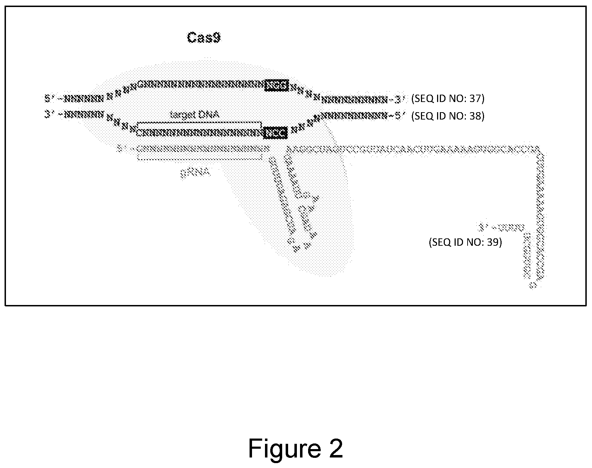

Figure 2 is a schematic representation of the Cas9/gRNA complex. The modified Cas9/gRNA complex (in yellow and grey, respectively) binds to a target DNA sequence and specifically cleaves within 3 nucleotides of the Protospacer Adjacent Motif NGG (black box).

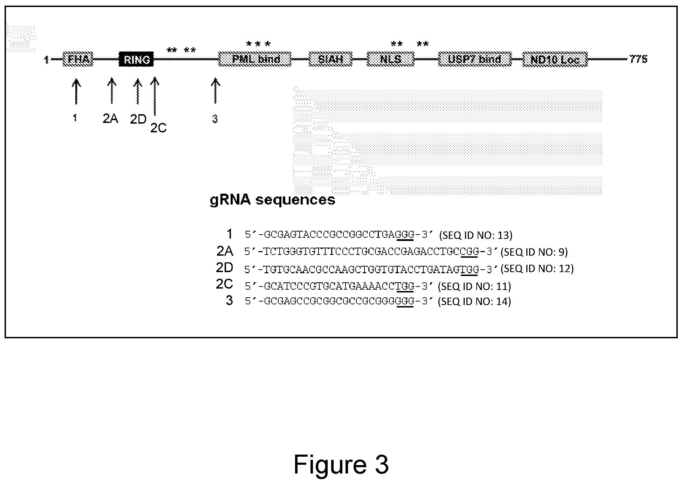

Figure 3 is a graphic representation of the protein sequence of ICPO, demonstrating domains [FHA (forkhead-associated phosphorylation motif), RING (Cys3His1Cys4 zinc binding) domain, PMHL binding domain, SIAH (seven in absentia homologue binding motif), NLS (nuclear localization sequence), USP7 (ubiquitin-specific proteinase 7-binding motif), and ND10 localization motif]. Asterisks indicate phosphorylation sites. The selected HSV1 targeted DNA (tDNA) sequences are indicated by arrows, with the full tDNA sequences shown beneath the graphic line along with their identifying names.

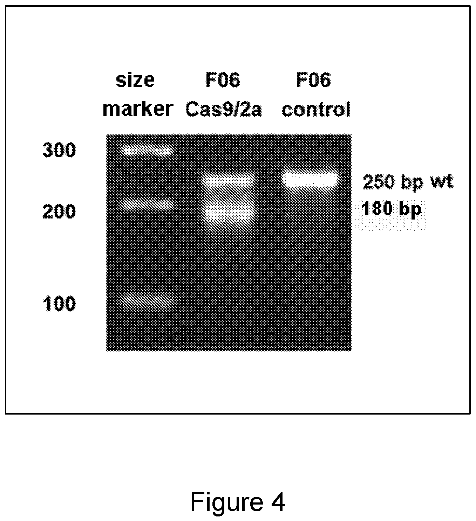

Figure 4 depicts an agarose gel of the surveyor assays of ICPO after stable transfection of F06 cells with Cas9/anti-HSV1 ICPO gRNA 2A. This demonstrates that a 180 base pair fragment is generated by this clone. The left lane shows molecular size markers. The right lane shows the untransfected F06 cells, which contain HSV1 ICPO genomic DNA.

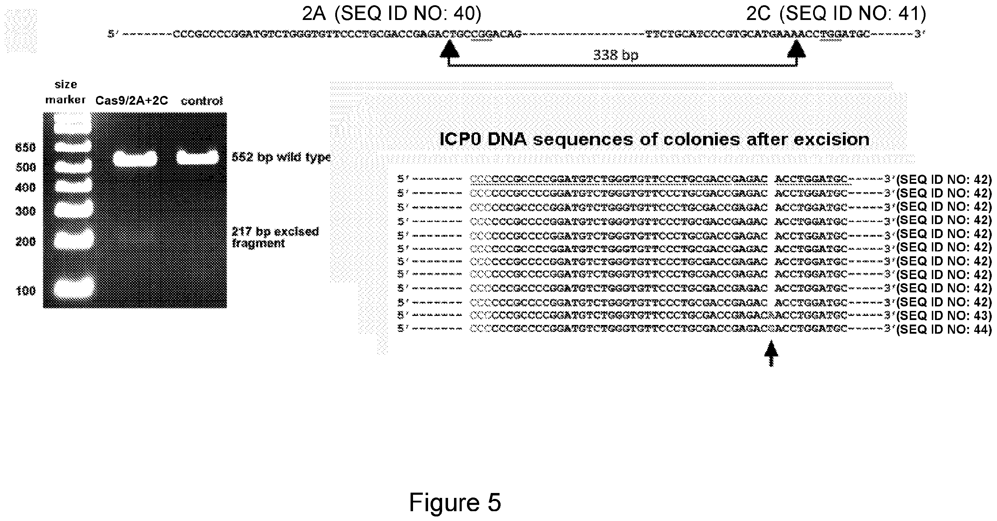

Figure 5 depicts the results of simultaneous transient coinfection of L7 cells with two of the Cas9/HSV1 gRNA sequences, Cas9/anti-ICP0 gRNA 2A and Cas9/anti-ICP0 gRNA 2C (abbreviated Cas9/2A+2C). The HSV1 native sequence of ICPO including the 2A and 2C tDNA sequences are shown at the top of Figure 5. The ten DNA sequences shown beneath are the sequences of the cloned resulting ICPO sequences, showing either a deletion of 1 base pair (the gap in the aligned sequence, which was observed in 8/10 sequenced clones) or an insertion of 1 base pair (2/10 clones). The coinfection of these two guide sequences resulted in cleavage of a 335bp portion of the ICPO sequence, leaving a 217 bp sequence of ICPO DNA. The inset shows the agarose gel demonstrating the cleaved 217 base pair cleaved DNA fragment (center lane). On the right is the untransfected L7 cells (aka no Cas9 constructs), demonstrating the full length wild type fragment alone. On the left are the molecular size markers.

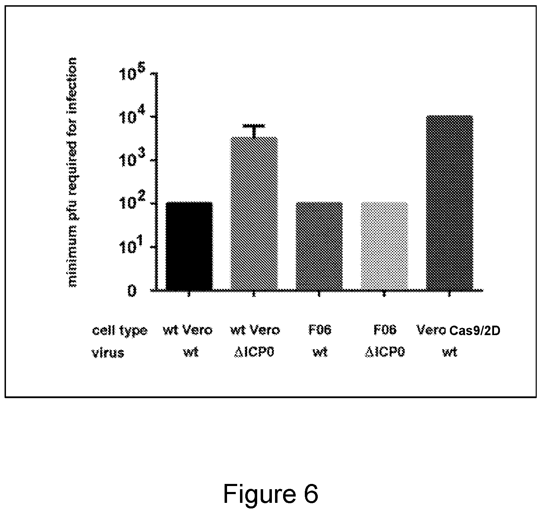

Figure 6 depicts the results of viral infection of cells after infection with Cas9/ anti-HSV1 ICPO gRNA 2D compared with controls. After stable transfection of normal Vero cells with Cas9/ anti-HSV1 ICPO gRNA, the cells become significantly more resistant to infection with HSV1, and cannot be infected with low concentrations of the virus. These results are equivalent to those seen when normal Vero cells were infected with mutant HSV1 virus that completely lacks ICPO (ΔICP0). When external ICPO is supplemented (the F06 cells express ICPO), the cells were infected with ICPO lacking HSV1 (ΔICP0) at significantly lower concentrations as expected. Pfu stands for plaque-forming units, which is a standard way to quantitate the amount of infectious HSV1 added to each condition in the experiment. Wt stands for wild type or regular herpes simplex virus, which contains ICPO DNA coding sequences.

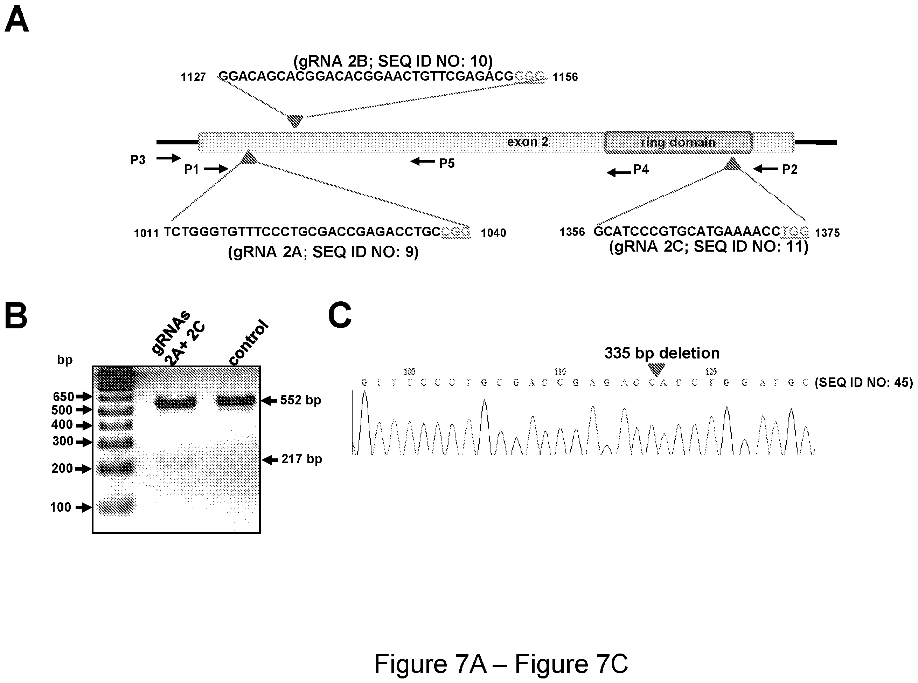

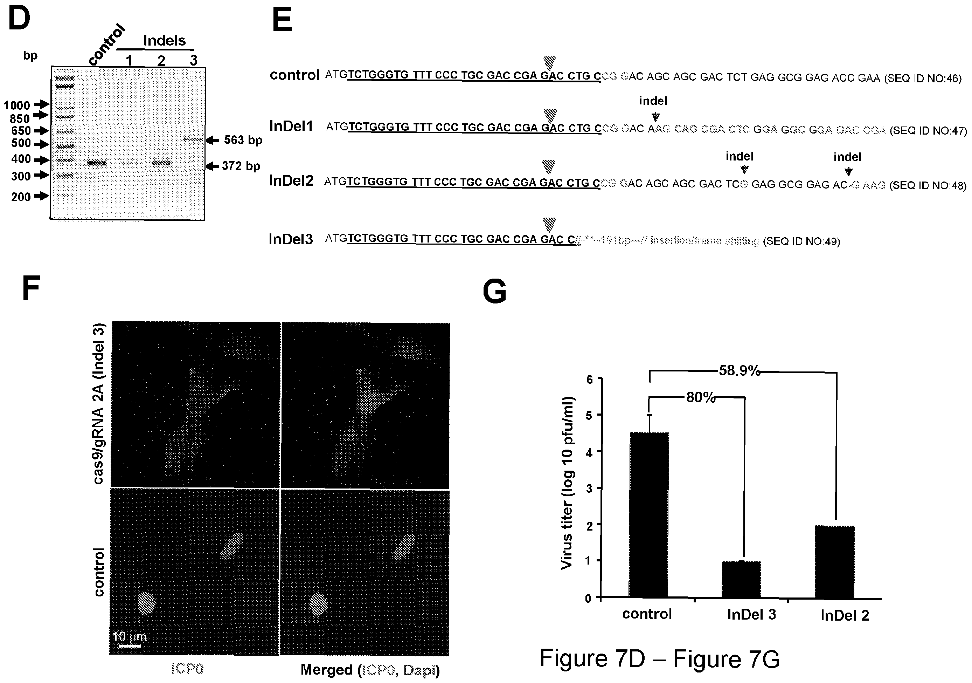

Figure 7, comprising Figure 7A through Figure 7G, depicts the results of experiments demonstrating that CRISPR/Cas9 introduces mutations in ICPO gene and reduces HSV-1 replication. Figure 7A: Schematic representation of HSV-1 ICPO genomic region showing exon II and the ring domain. The positions and nucleotide composition of 2A, 2B and 2C targets including PAM (marked in green) are shown. The positions of various primers (P) used in PCR amplification are illustrated. Figure 7B: Gel analysis of DNA fragments amplified by primers P3 and P2 (shown in Figure7A) in L7 cells expressing Cas9 (control) or Cas9 plus gRNAs 2A and 2C. The positions of an expected 552 bp amplicon and a smaller DNA fragment of 217 bp caused by cleavage of exon II DNA at targets 2A and 2C, and amplification of the remaining fragments after ligation are shown. Figure 7C: Sequence traces of the 217 bp cloned fragment (shown in Figure 7B) illustrating a blunt end-joining of the two ends of the cleaved exon II DNA after excision of 355 bp. Figure 7D: DNA gel analysis illustrating amplicons using P3 and P4 primers from several clonal cells (named InDels 1-3) expressing CAs9/gRNA 2A or the control cells with no gRNA 2A. Figure 7E: DNA sequencing identified nucleotide insertions (as depicted by black arrowheads) in clones InDel 1 and 2, and insertion of 191 bp DNA in clones InDel 3. The red arrowhead points to the cleavage sites. Figure 7F: Representative confocal images of L7 cells (control) and its subclones InDel 3 (shown in Figure 7E) expressing Cas9/gRNA 2A after immunostaining with an antibody against ICPO. Green is indicative of expression of GFP by HSV-1 and DAPI (blue) depicts the nuclei of the cells. Figure 7G: Viral production assay demonstrating the titer of ΔICP0-HSV-1 in the supernatant of parental L7 cells (control) endogenously expressing ICPO or in InDel 3, InDel 2 (shown in Figure 7E).

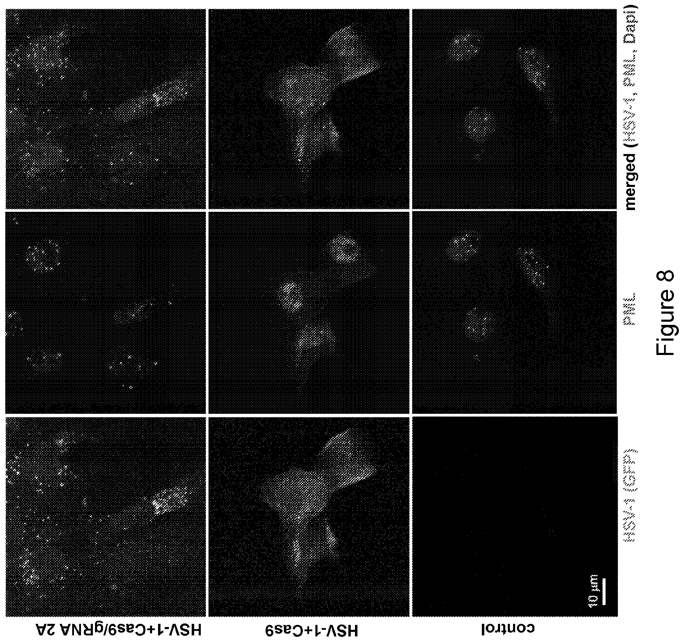

Figure 8 depicts the results of experiments demonstrating that CRISPR/Cas9 by targeting ICPO expression restores association of PML bodies in L7 cells. Immunostaining analysis of PML bodies in control, uninfected L7 cells shows the appearance of punctate staining of PML bodies within the nuclei of the cells (lower panel). DAPI staining of nuclei (blue) is shown in the right panels. Infection of Vero L7 cells expressing Cas9 at low MOI with HSV-1/GFP shows complete destruction of the PML and robust replication of HSV-1/GFP as shown in green (middle panels). In L7 cells expressing Cas9/gRNA 2A infection with HSV-1/GFP failed to disrupt formation of punctate PML in the nuclei and significantly reduced the level of viral replication detected in the cells (green, top panels).

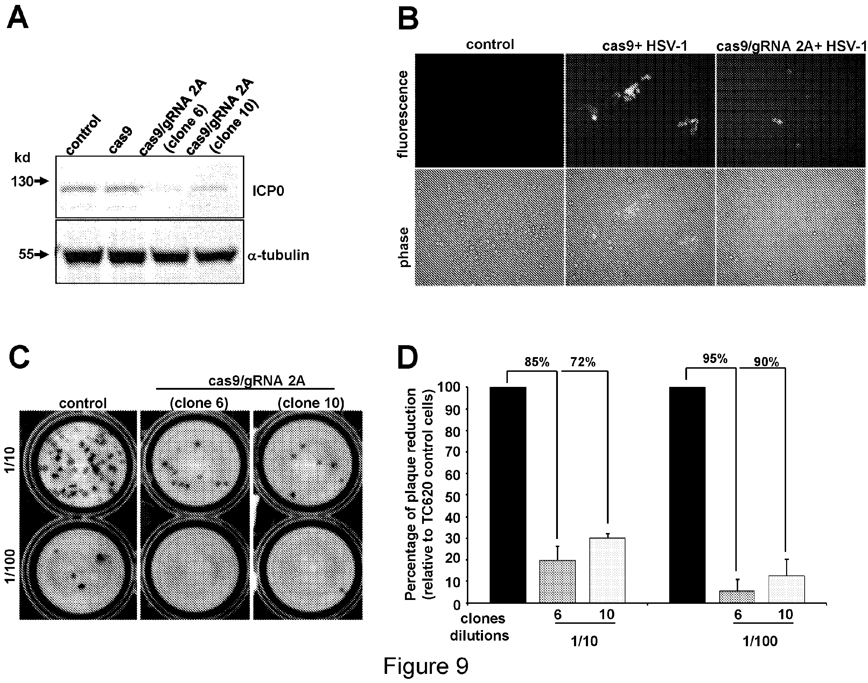

Figure 9, comprising Figure 9A through Figure 9D, depicts the results of experiments demonstrating that human TC620 cells expressing Cas9/gRNAs do not support HSV-1 replication. Figure 9A: Western blot analysis of protein extracts for detection of ICPO in TC620 human olidodendroglioma cells and its subclones expressing Cas9 or Cas9/gRNA 2A (clones 6 and 10). Expression of 110 kDa ICPO is detected in the control cells and cells expressing Cas9, but severely reduced in clones 6 and 10. Expression of a housekeeping protein, α-tubulin is shown. Figure 9C: Representative plaque assay using the supernatant from HSV-1/GFP infected control TC620 or clones 6 and 10 at two different dilutions showing a drastic decrease in the number of plaques as a result of suppression of ICPO by Cas9/gRNA editing of the infected cells. Figure 9D: Quantification of HSV-1 production by plaque assay shows drastic suppression of the viral production in TC620 cells with continuous production of Cas9 plus gRNA 2A.

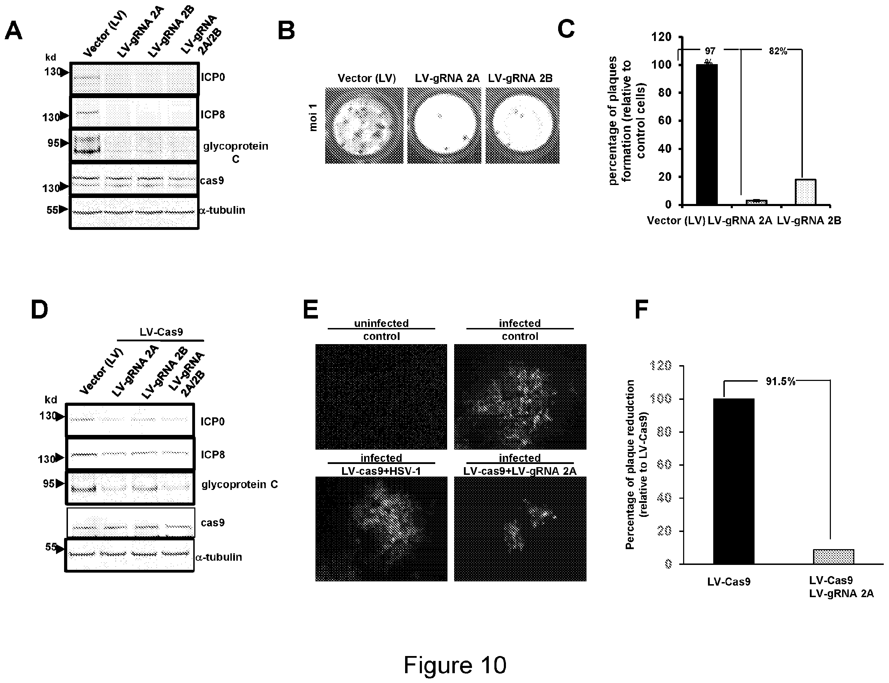

Figure 10 comprising Figure 10A through Figure 10F, depicts the results of experiments demonstrating that lentivirus (LV) mediated Cas9/gRNA delivery suppresses HSV-1 infection during the lytic cycle and prevents uninfected cells from new infection. Figure 10A: Western blot analysis of protein extracts from TC260 cells that endogenously express Cas9 and are infected with HSV-1/GFP for 24 hours and thereafter treated with lentivirus expressing gRNAs 2A, 2B or both and harvested 48 hours later for protein extraction. The positions of ICPO, ICP8, glycoprotein C, Cas9 and α-tubulin are shown. Figure 10B and Figure 10C illustrate results from plaque assay in which cells treated with LVgRNA 2A or LVgRNA 2B showed a drastic decrease in virus production. Figure 10D: Western blot analysis of protein extracts from the infected cells as described above. Figure 10E: Confocal immunofluorescent evaluation of HSV-1/GFP replication in TC620 cells that were treated with vector (LV) LVCas9, LVCas9 plus LVgRNA 2A, LVCas9 plus gRNA 2B and LVCas9 plus LVgRNA 2A and 2B at 48 hours prior to HSV-1 infection. A decrease in the replication of HSV-1/GFP is shown by examination of green fluorescence, which demonstrates fewer HSV-1 infected cells in LV-Cas9+LV-gRNA 2A treated cells.. Figure 10F: Plaque assay showing a decrease in the titer of the virus released upon HSV-1 infection of cells that were pre-treated with LV producing Cas9 and gRNAs as depicted.

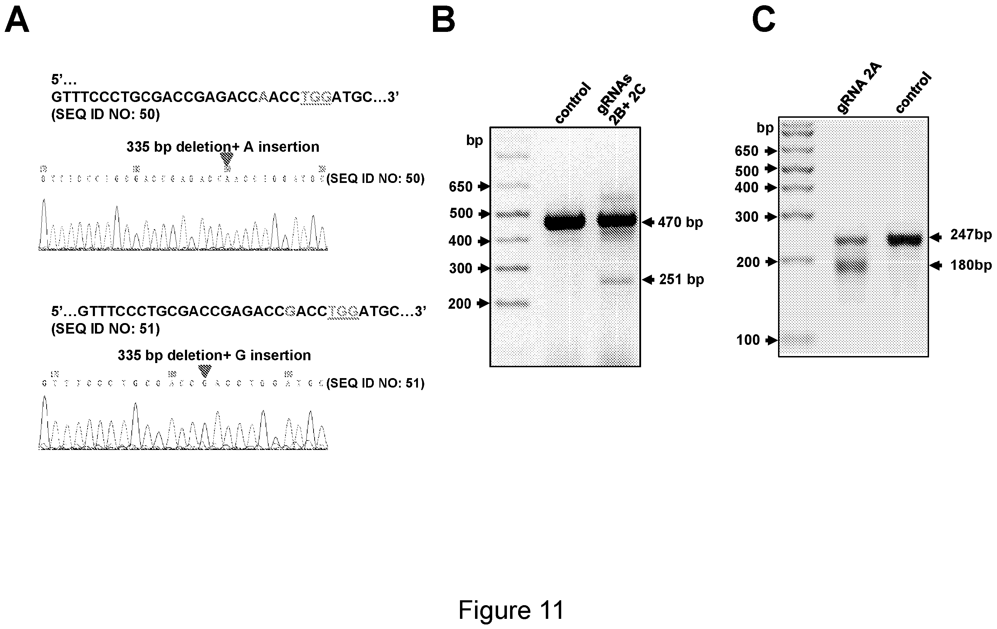

Figure 11, comprising Figure 11A through Figure 11C depicts the results of example experiments.. Figure 11A: Chromatograms showing the detection of breakpoints within exon II as a result of cleavage of DNA by Cas9 and gRNAs 2A and 2C, and insertion of a single nucleotide A (top) and G (bottom) at the site of the breakpoint. Figure 11B: Recombination of gRNA 2B and 2C cuts within exon II generating a new template for amplification of a 251 bp DNA. Figure 11C: SURVEYOR assay showing InDel mutation in exon II at target A causing a new DNA fragment of 180 bp after cleavage with Cel1 nuclease (in lane marked gRNA 2A).

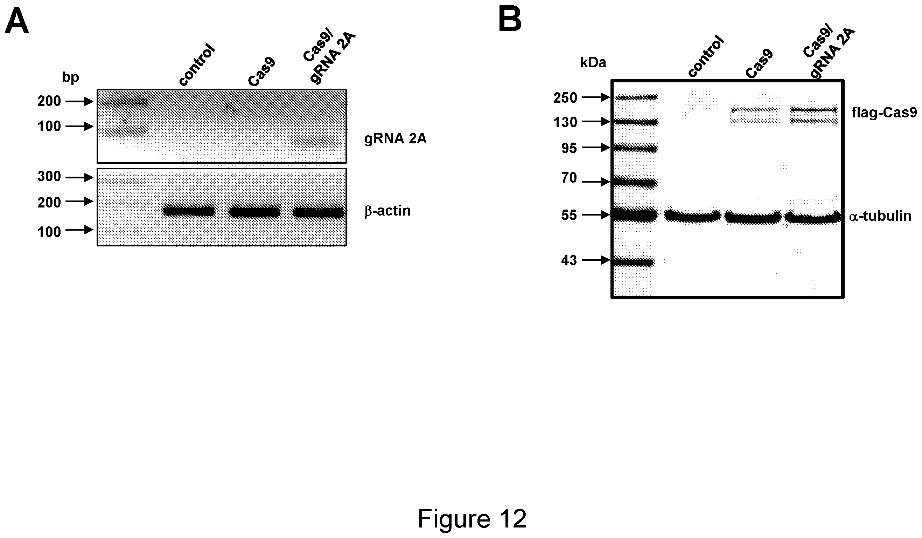

Figure 12, comprising Figure 12A and Figure 12B, depicts the results of example experiments demonstrating production of Cas9 gRNA and Cas9 protein in treated human cells. Figure 12A: Production of gRNA 2A in human TC620 cells either not treated (control) expressing Cas9, or Cas9 plus gRNA 2A by RT-PCR. β-actin transcripts served as a loading control. Figure 12B: Expression of flag-Cas9 in TC620 cells were verified by Western blot analysis.

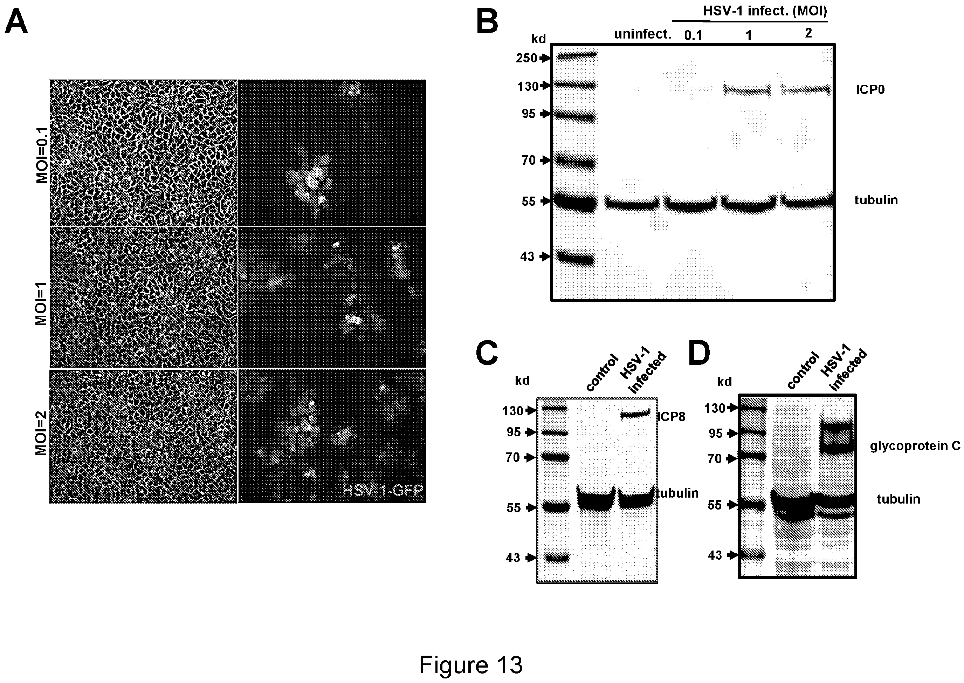

Figure 13, comprising Figure 13A through Figure 13D, depicts the results of example experiments demonstrating the effective infection of human cell line TC620 with HSV0-1. Figure 13A: TC620, a human olidodendroglioma cell line, were infected with HSV-1/GFP at MOI of 0.1, 1 or 2, and 48 hours later, expression of GFP, indicative for HSV-1 expression and replication, was assessed by fluorescence microscopy. Figure 13B: Western blot analysis for evaluating expression of ICPO in TC620 cells infected with HSV-1 at 48 hours post-infection. Figure 13C and Figure 13D: Detection of ICP8 and glycoprotein C in infected TC620 cells at MOI=1, 48 hours post-infection by Western blot analysis.

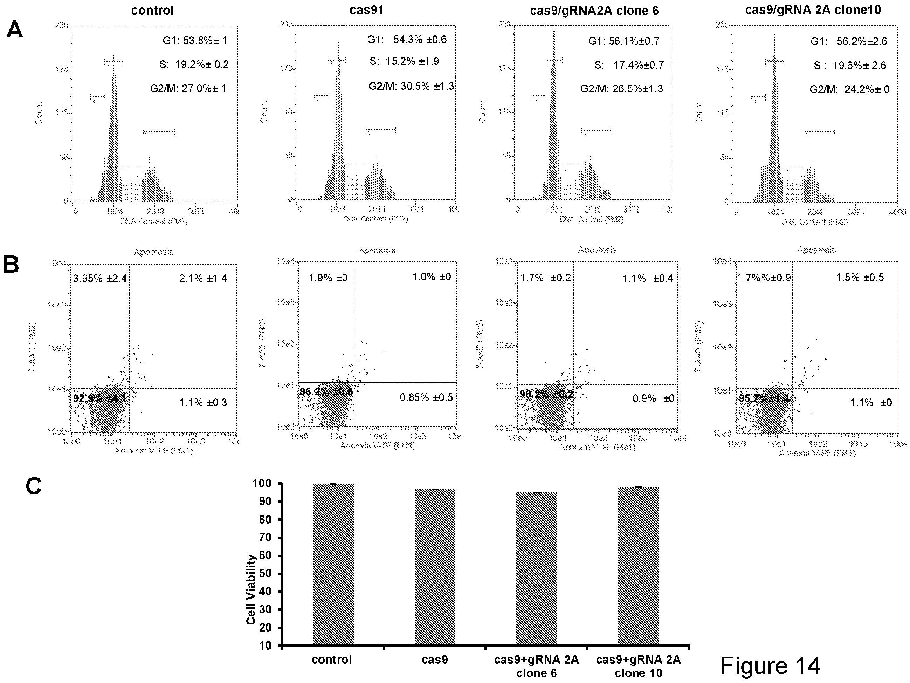

Figure 14, comprising Figure 14A through Figure 14C, depicts the results of example experiments demonstrating that treatment of human cells with Cas9/gRNAs does not impact cell cycle, apoptosis, or cell survival. Figure 14A: Cell cycle assay was used to investigate the impact of Cas9/gRNA on the cell cycle of TC620 control cells has no expression of Cas9 or any gRNAs, whereas subclones of Cas9 and Cas9/gRNA 2A clones 6 and 10 express either Cas9 alone or Cas9 plus gRNA2A as indicated. As seen, no differences in cell cycle progression through G1, S and G2/M phases were detected in these cell lines. Figure 14B: Apoptotic assay in the clonal cell lines as shown in Figure 14A showed no significant changes in annexin stained cell population (ranging from 1.0-2.1%). Figure 14C: Cell viability assay using propidium iodide staining assay showed changes in the viability of the cells with Cas9 or Cas9/gRNA expression in the various clones.

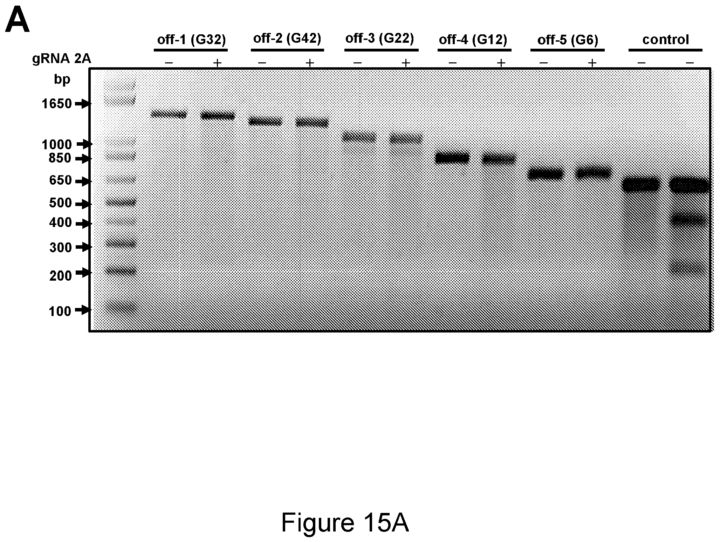

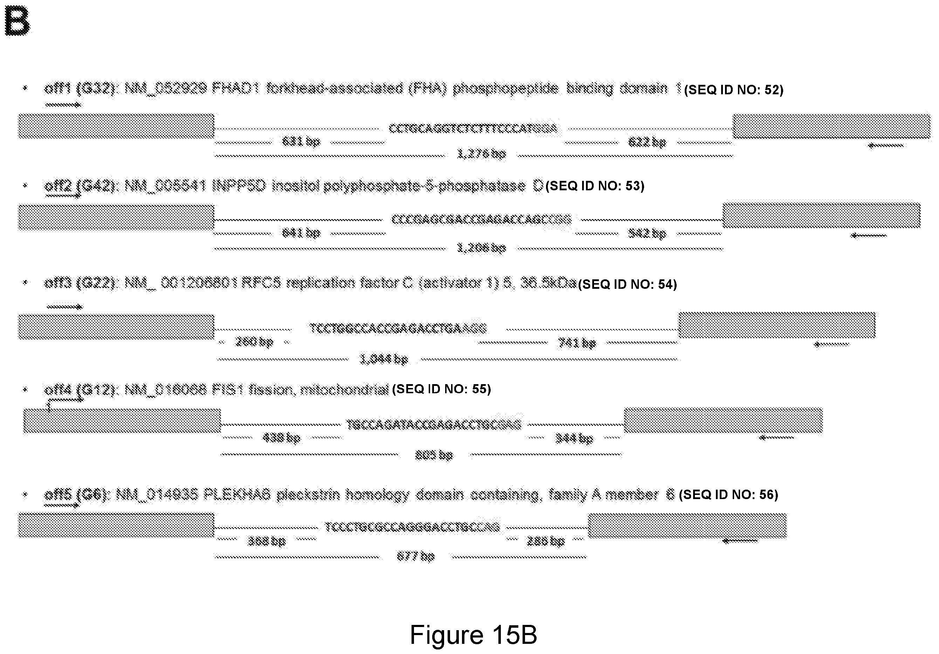

Figure 15, comprising Figure 15A and Figure 15B, depicts the results of example experiments demonstrating that production of Cas9/gRNA does not cause changes in host cell DNA. Figure 15A: DNA gel analysis of SURVEYOR assay illustrating no detectable bands below the expected DNA fragment from PCR amplification that can be attributed to Cas9/gRNA 2A expression. Control is provided by the supplier of the SURVEYOR assay kit (Integrated DNA Technologies, Coralville, IA). Figure 15B. Results from PCR amplification and Sanger sequencing of the cellular genes that contained potential off-targets (as shown in Figure 15A) revealed no InDel mutations in the amplicons. Arrows demonstrate the positions of the primers and their distance from the potential off-targets. Red indicates the PAM sequence and the black illustrate the potential target sequence for each cellular gene. The size of each amplicon is indicated.

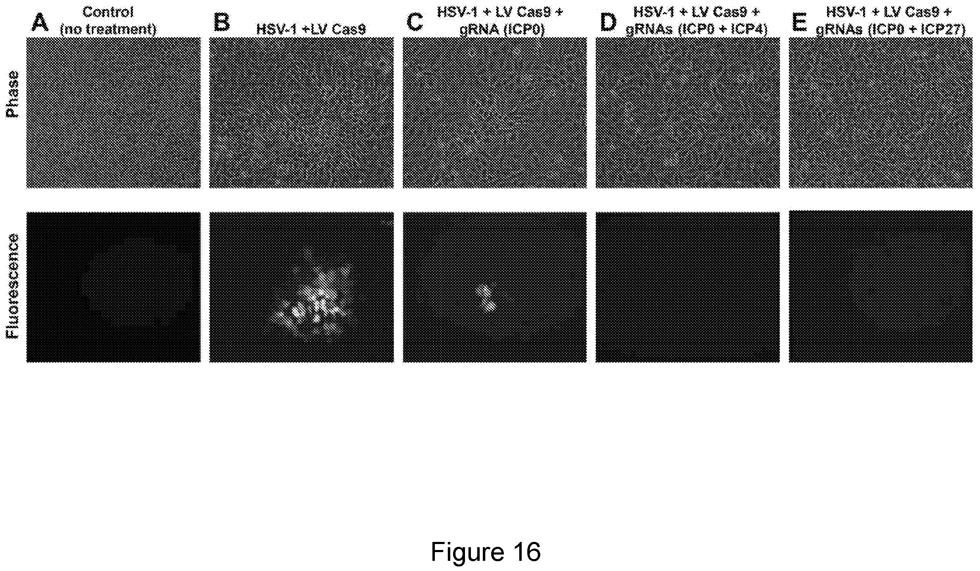

Figure 16, comprising Figure 16A through Figure 16E, depicts the results of example experiments. 6. Figure 16A - Figure 16E. Epifluorescent assessment of HSV-1/GFP replication in TC260 cells after treatment with lentivirus vector (LV) expressing Cas9 or various gRNAs as shown above each panel according to the method described for Figure 10E. Images are representative of duplicate plates for each condition.

### DETAILED DESCRIPTION

The present invention relates to compositions for use in the treatment or prevention of a herpesvirus infection in a subject in need thereof. For example, in certain embodiments, the present invention provides a composition that specifically cleaves target sequences in human alpha-herpesviruses, for example, human herpes simplex type 1 (HSV1). Such compositions, which can include a Clustered Regularly Interspaced Short Palindromic Repeats (CRISPR) nucleic acid or polypeptide and specific guide RNA sequences, can be administered to a subject having acute or latent alpha-herpesvirus infections.

### Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

As used herein, each of the following terms has the meaning associated with it in this section.

The articles "a" and "an" are used herein to refer to one or to more than one (*i.e.,* to at least one) of the grammatical object of the article. By way of example, "an element" means one element or more than one element.

"About" as used herein when referring to a measurable value such as an amount, a temporal duration, and the like, is meant to encompass variations of ±20%, ±10%, ±5%, ±1%, or ±0.1% from the specified value, as such variations are appropriate to perform the disclosed methods.

The term "abnormal" when used in the context of organisms, tissues, cells or components thereof, refers to those organisms, tissues, cells or components thereof that differ in at least one observable or detectable characteristic (e.g., age, treatment, time of day, etc.) from those organisms, tissues, cells or components thereof that display the "normal" (expected) respective characteristic. Characteristics which are normal or expected for one cell or tissue type, might be abnormal for a different cell or tissue type.

A "disease" is a state of health of an animal wherein the animal cannot maintain homeostasis, and wherein if the disease is not ameliorated then the animal's health continues to deteriorate.

In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

A disease or disorder is "alleviated" if the severity of a symptom of the disease or disorder, the frequency with which such a symptom is experienced by a patient, or both, is reduced.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

An "effective amount" or "therapeutically effective amount" of a compound is that amount of compound which is sufficient to provide a beneficial effect to the subject to which the compound is administered. An "effective amount" of a delivery vehicle is that amount sufficient to effectively bind or deliver a compound.

"Expression vector" refers to a vector comprising a recombinant polynucleotide comprising expression control sequences operatively linked to a nucleotide sequence to be expressed. An expression vector comprises sufficient cisacting elements for expression; other elements for expression can be supplied by the host cell or in an in vitro expression system. Expression vectors include all those known in the art, such as cosmids, plasmids (*e.g*., naked or contained in liposomes) and viruses (*e.g.*, lentiviruses, retroviruses, adenoviruses, and adeno-associated viruses) that incorporate the recombinant polynucleotide.

"Homologous" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared X 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

"Isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

The terms "patient," "subject," "individual," and the like are used interchangeably herein, and refer to any animal, or cells thereof whether *in vitro* or *in situ,* amenable to the methods described herein. In certain non-limiting embodiments, the patient, subject or individual is a human.

"Parenteral" administration of a composition includes, *e.g*., subcutaneous (s.c.), intravenous (i.v.), intramuscular (i.m.), or intrasternal injection, or infusion techniques.

The term "polynucleotide" as used herein is defined as a chain of nucleotides. Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR^{™}, and the like, and by synthetic means.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

The term "promoter" as used herein is defined as a DNA sequence recognized by the synthetic machinery of the cell, or introduced synthetic machinery, required to initiate the specific transcription of a polynucleotide sequence.

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

A "constitutive" promoter is a nucleotide sequence which, when operably linked with a polynucleotide which encodes or specifies a gene product, causes the gene product to be produced in a cell under most or all physiological conditions of the cell.

An "inducible" promoter is a nucleotide sequence which, when operably linked with a polynucleotide which encodes or specifies a gene product, causes the gene product to be produced in a cell substantially only when an inducer which corresponds to the promoter is present in the cell.

A "tissue-specific" promoter is a nucleotide sequence which, when operably linked with a polynucleotide encodes or specified by a gene, causes the gene product to be produced in a cell substantially only if the cell is a cell of the tissue type corresponding to the promoter.

A "therapeutic" treatment is a treatment administered to a subject who exhibits signs of pathology, for the purpose of diminishing or eliminating those signs.

As used herein, "treating a disease or disorder" means reducing the frequency with which a symptom of the disease or disorder is experienced by a patient. Disease and disorder are used interchangeably herein.

The phrase "therapeutically effective amount," as used herein, refers to an amount that is sufficient or effective to prevent or treat (delay or prevent the onset of, prevent the progression of, inhibit, decrease or reverse) a disease or condition, including alleviating symptoms of such diseases.

To "treat" a disease as the term is used herein, means to reduce the frequency or severity of at least one sign or symptom of a disease or disorder experienced by a subject.

"Variant" as the term is used herein, is a nucleic acid sequence or a peptide sequence that differs in sequence from a reference nucleic acid sequence or peptide sequence respectively, but retains essential properties of the reference molecule. Changes in the sequence of a nucleic acid variant may not alter the amino acid sequence of a peptide encoded by the reference nucleic acid, or may result in amino acid substitutions, additions, deletions, fusions and truncations. Changes in the sequence of peptide variants are typically limited or conservative, so that the sequences of the reference peptide and the variant are closely similar overall and, in many regions, identical. A variant and reference peptide can differ in amino acid sequence by one or more substitutions, additions, deletions in any combination. A variant of a nucleic acid or peptide can be a naturally occurring such as an allelic variant, or can be a variant that is not known to occur naturally. Non-naturally occurring variants of nucleic acids and peptides may be made by mutagenesis techniques or by direct synthesis.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, and the like.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The present invention provides compositions for use in treating and preventing a herpesvirus infection in a subject in need thereof. For example, in certain embodiments, the present invention provides a composition that specifically cleaves target sequences in the viral genome of a herpesvirus, thereby preventing or reducing the ability of the virus to replicate and thus inhibiting herpesvirus infectivity.

The present invention is based, in part, on results obtained using a CRISPR-Cas9 system in single and multiplex configurations specific to the human herpes simplex type I virus (also referred to as HSV1). The CRISPR-Cas9 system used in experiments presented elsewhere herein compromised the integrity of the viral DNA sequences corresponding to the first two coding exons of essential HSV1 proteins by introducing insertion/deletion (InDel) mutations. It is demonstrated herein that CRISPR-Cas9 mediated HSV1-specific gene editing results in the inactivation of viral gene expression and slows or stops viral replication in cells. For example, the CRISPR-Cas9 molecules described herein have the potential to remove a large segment of the HSV1 genome and cripple the ability of the virus to replicate in infected cells. Furthermore, the data presented herein demonstrate that the preexisting presence of HSV1-directed Cas9 in cells immunized them against HSV1 infection. For example, the RNA-guided Cas9 presented herein acts as molecular scissors and, by disrupting various regions of the HSV1 genome, abrogates replication of the virus. Thus, the present invention provides a composition and method that targets the HSV1 genome in infected cells for destruction of the viral genome in acute or latent HSV1 infection as a novel therapeutic and prophylactic strategy.

In one embodiment, the present invention provides a composition for use in the treatment or prevention of a herpesvirus infection in a subject in need thereof. In one embodiment, the composition comprises at least
(a) an isolated nucleic acid encoding a CRISPR-associated (Cas) peptide; and
(b) one or more isolated nucleic acids, wherein the one or more isolated nucleic acids encode multiple guide nucleic acids, wherein each guide nucleic acid comprises a nucleotide sequence complementary to a different target sequence in the herpesvirus genome, and wherein a guide nucleic acid of the multiple guide nucleic acids comprises a target sequence complementary to a sequence within the ICPO domain of the herpesvirus genome and wherein a second guide nucleic acid of the multiple guide nucleic acids comprises a target sequence complementary to a sequence within the ICP4 or ICP27 domain of the herpesvirus genome. Together, the nucleic acid guide molecule and the CRISPR-associated (Cas) peptide function to introduce one or more mutations at target sites within the herpesvirus genome, which thereby inhibits the infectivity of the virus.

The composition thus encompasses isolated nucleic acids encoding one or more elements of the CRISPR-Cas system. For example, in one embodiment, the composition comprises an isolated nucleic acid encoding at least one of the guide nucleic acid molecule and a CRISPR-associated (Cas) peptide, or functional fragment or derivative thereof.

The present invention is useful in a method for the treatment or prevention of a herpesvirus infection in a subject in need thereof. This method comprises administering to the subject an effective amount of a composition discussed above. In certain instances the method comprises administering a composition comprising an isolated nucleic acid encoding at least one of the guide nucleic acid molecule and a CRISPR-associated (Cas) peptide, or functional fragment or derivative thereof. The method can comprise administering a composition described herein to a subject diagnosed with a herpesvirus infection, at risk for developing a herpesvirus infection, a subject with a latent herpesvirus infection, and the like.

This method is used to treat or prevent a herpesvirus infection, including but not limited to herpes simplex type I (HSV1), herpes simplex virus 2 (HSV2), human herpresvirus-3 (HHV-3; varicella zoster virus (VZV), human herpesvirus-4 (HHV-4; Epstein-Barr virus (EBV)), human herpesvirus-5 (HHV-5; Cytomegalovirus (CMV)), human herpesvirus-6 (HHV-6; roseolovirus), human herpes virus-7 (HHV-7), and human herpesvirus-8 (HHV-8; Karposi's sarcoma-associated herpesvirus (KSHV)).

### Herpesvirus

The herpesvirus genus is divided into three genera: alpha-herpesviruses (e.g., HSV1, herpes simplex type 2 which causes genital herpes, and varicella-zoster virus which causes chickenpox and shingles); beta-herpesviruses (e.g. HHV-6 which causes sixth disease, and HHV-7, which causes roseola infantum); and gammaherpesviruses (e.g. Epstein-Barr virus which causes mononucleosis and other disease, and HHV-8 which causes Kaposi's sarcoma). The alpha-herpesviruses not only share a similar lifecycle but also have homologous DNA sequences in many of the viral proteins that are essential for viral replication and reactivation.

Herpes simplex type 1 (HSV1) is an encapsulated, double-stranded 153 kilobase (kB) DNA virus that is a nearly ubiquitous human pathogen. Up to 60% of the U.S. population has been infected with HSV1 by their 40s. Primary infection typically occurs in early childhood and is characterized by fever and lesions of the buccal and gingival mucosa, although subclinical infection frequently occurs. Primary HSV1 infection can also occur through sexual contact, and HSV1 is increasing found to be the cause of genital herpes. While symptoms of the primary infection are typically fairly mild, life-threatening infections can occur, particularly if the infection is incurred in the neonatal period or if HSV1 is contacted by immunodeficient individuals.

Following primary infection, the HSV1 genome can lie dormant in sensory neurons for an extended period of time. During this stage of the viral lifecycle, the HSV1 genome is present within the nucleus of latently infected neurons in a supercoiled, episomal state. In this state, no viral proteins are produced, and only one viral transcript, the latency-associated transcript (LAT) is produced. A number of stimuli, including stress and UV light, can lead to viral reactivation from latently infected neurons. Reactivation can occur repeatedly many years after the initial infection. Symptoms caused by HSV1 reactivation vary by the site of primary infection and extent of reactivation. The most commonly recognized form of HSV1 reactivation is cold sores. These typically appear on the vermilion border of the mouth following a variety of stimuli, including UV light exposure. Reactivation of virus stored in dorsal root ganglion neurons innervating the genitalia takes the form of recurrent genital herpes. Orolabial and anogenital manifestations of HSV1 reactivation are marked by an initial prodromal tingling in the area followed by the emergence of painful blisters containing infectious virus that ulcerate and ultimately heal. Other, more uncommon manifestations of HSV1 reactivation include Bell's palsy, delayed facial palsy after otologic surgery, and vestibular neuritis. HSV1 reactivation can have more devastating manifestations, such as herpes encephalitis and disseminated herpes.

During primary infection, a defined sequence of protein expression occurs (Figure 1). Following viral entry into cells during primary infection, the viral capsid is released within the cytoplasm and host protein synthesis is shut down by VHS/ULA41, a tegument protein. A second tegument protein, VP16, forms a complex with host proteins Oct1 and HCF to induce immediate early HSV1 transcription. These immediate early genes induce the expression of HSV1 encoded enzymes required for viral DNA replication, including thymidine kinase (TK) and the viral DNA polymerase (UL30). As viral DNA production progresses, the late viral proteins including capsid proteins, tegument proteins, and glycoproteins necessary for viral entry into cells are produced. Viral particles assemble and viral DNA is packaged into capsids. Ultimately, infectious viral particles are produced, leading to infection of surrounding cells and allowing transmission to other people.

The sequence of initial steps of viral reactivation is thought to be echoed during reactivation of HSV1 in latently infected neurons at later stages of reactivation. Proteins which play key roles in lytic infection, such as VP16, are thought to play similar critical roles in reactivation. As the process of HSV1 reactivation progresses, immediate early gene expression occurs followed by early and then late protein production. Ultimately the production of infectious viral particles occurs, leading to transmission of infection to neighboring cells and uninfected individuals.

In recent years, several systems for targeting endogenous genes have been developed including homing endonucleases (HE) or meganucleases, zinc finger nucleases (ZFN), transcription activator-like effector nucleases (TALEN) and most recently clustered regularly interspaced short palindromic repeats (CRISPR)-associated system 9 (Cas9) proteins which utilize site-specific double-strand DNA break (DSB)-mediated DNA repair mechanisms. These enzymes induce a precise and efficient genome cutting through DSB-mediated DNS repair mechanisms. These DSB-mediated genome editing techniques enable target gene deletion, insertion, or modification.

In the past years, ZFNs and TALENs have revolutionized genome editing. The major drawbacks for ZFNs and TALENs are the uncontrollable off-target effects and the tedious and expensive engineering of custom DNA-binding fusion protein for each target site, which limit the universal application and clinical safety.

The RNA-guided Cas9 biotechnology induces genome editing without detectable off-target effects. This technique takes advantage of the genome defense mechanisms in bacteria that CRISPR/Cas loci encode RNA-guided adaptive immune systems against mobile genetic elements (viruses, transposable elements and conjugative plasmids). Three types (I-III) of CRISPR systems have been identified. CRISPR clusters contain spacers, the sequences complementary to antecedent mobile elements. CRISPR clusters are transcribed and processed into mature CRISPR (Clustered Regularly Interspaced Short Palindromic Repeats) RNA (crRNA). Cas9 belongs to the type II CRISPR/Cas system and has strong endonuclease activity to cut target DNA.

Cas9 is guided by a mature crRNA that contains about 20 base pairs (bp) of unique target sequence (called spacer) and a trans-activated small RNA (tracrRNA) that serves as a guide for ribonuclease III-aided processing of pre-crRNA. The crRNA:tracrRNA duplex directs Cas9 to target DNA via complementary base pairing between the spacer on the crRNA and the complementary sequence (called the protospacer) on the target DNA (tDNA). Cas9 recognizes a trinucleotide (NGG) protospacer adjacent motif (PAM) to specify the cut site (the 3^{rd} nucleotide from PAM). The crRNA and tracrRNA can be expressed separately or engineered into an artificial fusion small guide RNA (gRNA) via a synthetic stem loop (AGAAAU) to mimic the natural crRNA/tracrRNA duplex. Such gRNA, like shRNA, can be synthesized or in vitro transcribed for direct RNA transfection or expressed from a RNA expression vector (e.g., U6 or H1 promoter-driven vectors). Therefore, the Cas9 gRNA technology requires the expression of the Cas9 protein and gRNA, which then form a gene editing complex at the specific target DNA binding site within the target genome and inflict cleavage/mutation of the target DNA.

However, the present invention is not limited to the use of Cas9-mediated gene editing. Rather, the present invention encompasses the use of other CRISPR-associated peptides, which can be targeted to a targeted sequence using a gRNA and can edit to target site of interest. For example, in one embodiment, the invention utilizes Cpf1 to edit the target site of interest.

As described herein, in one embodiment, the present invention employs a genetic strategy using a novel RNA-guided CRISPR technology that targets the HSV1 genome and by editing the specific domain of the HSV1 immediate early gene, infected cell protein 0 (ICPO), abrogates its expression.

However, the present invention is not limited to the prevention or treatment of HSV1. Rather, the present invention may be used to treat or prevent other herpesviruses. For example, as the HSV2 genome is similar to the HSV1 genome, the strategy described herein would be effective in treating or preventing HSV2.

### Composition

The present invention provides a composition for the treatment or prevention of a herpesvirus infection in a subject in need thereof. In certain aspects the composition comprises one or more isolated nucleic acids encoding the CRISPR/Cas gene editing.

### Guide nucleic acid molecule

In one embodiment, the composition comprises nucleic acids encoding at least one guide nucleic acid molecule, or fragment thereof, where the guide nucleic acid molecule comprises a nucleotide sequence that is complementary to one or more target sequences in the herpesvirus genome. In one embodiment the guide nucleic acid is a guide RNA (gRNA).

In one embodiment, the gRNA comprises a crRNA:tracrRNA duplex. In one embodiment, the gRNA comprises a stem-loop that mimics the natural duplex between the crRNA and tracrRNA. In one embodiment, the stem-loop comprises a nucleotide sequence comprising AGAAAU. For example in one embodiment, the composition comprises a synthetic or chimeric guide RNA comprising a crRNA, stem, and tracrRNA.

In certain embodiments, the composition comprises an isolated crRNA and/or an isolated tracrRNA which hybridize to form a natural duplex. For example, in one embodiment, the gRNA comprises a crRNA or crRNA precursor (pre-crRNA) comprising a targeting sequence.

In one embodiment, the gRNA comprises a nucleotide sequence that is complementary to a target sequence in the herpesvirus genome. The target sequence in the herpesvirus genome may be any sequence in any coding or non-coding region where CRISPR/Cas-mediated gene editing would result in the mutation of the genome and inhibition of viral infectivity. The target sequence, to which the gRNA is complementary, is within the ICPO, ICP4, or ICP27 genes.

In certain embodiments the sequence of the gRNA that is complementary to the target is about 10-30 nucleotides in length. In certain embodiments, the gRNA comprises a nucleotide sequence that binds to the target sequence of the HSV genome. For example, in certain embodiments, the gRNA comprises a nucleotide sequence that is complementary to the target sequence, and thus binds to the target sequence. For example, exemplary nucleotide sequences which may be used to target the gRNA to the target sequence in the genome is provided in Figure 3, Figure 7, Example 1 and Example 2.

For example, in certain embodiments, the gRNA is complementary to a target sequence of the HSV genome selected from the group consisting of:
TCTGGGTGTTTCCCTGCGACCGAGACCTGC (SEQ ID NO: 1, "2A");
GGACAGCACGGACACGGAACTGTTCGAGACG (SEQ ID NO: 2, "2B")
GCATCCCGTGCATGAAAACC (SEQ ID NO: 3, "2C");
TGTGCAACGCCAAGCTGGTGTACCTGATAG-3' (SEQ ID NO: 4, "2D");
GCGAGTACCCGCCGGCCTGA (SEQ ID NO: 5, "1");
GCGAGCCGCGGCGCCGCGGG (SEQ ID NO: 6, "3");
TTCTACGCGCGCTATCGCGA (SEQ ID NO: 7, "ICP4 m1")
GGAGTGTTCCTCGTCGGACG (SEQ ID NO: 8, "ICP27 m1")

In certain embodiments, the target sequence precedes PAM sequence. For example, in one embodiment the target sequence precedes a NGG PAM sequence. Exemplary target sequences + PAM sequences (PAM sequences being underlined) are as follows:
TCTGGGTGTTTCCCTGCGACCGAGACCTGCCGG (SEQ ID NO: 9, "2A");
GGACAGCACGGACACGGAACTGTTCGAGACGGGG (SEQ ID NO: 10, "2B")
GCATCCCGTGCATGAAAACCTGG (SEQ ID NO: 11, "2C");
TGTGCAACGCCAAGCTGGTGTACCTGATAGTGG (SEQ ID NO: 12, "2D");
GCGAGTACCCGCCGGCCTGAGGG (SEQ ID NO: 13, "1");
GCGAGCCGCGGCGCCGCGGGGGG (SEQ ID NO: 14, "3");
TTCTACGCGCGCTATCGCGACGG (SEQ ID NO: 15, "ICP4 m1")
GGAGTGTTCCTCGTCGGACGAGG (SEQ ID NO: 16, "ICP27 m1")

Further, the invention encompasses an isolated nucleic acid (e.g., gRNA) having substantial homology to a nucleic acid disclosed herein. In certain embodiments, the isolated nucleic acid has at least 75%, 80%, 85%, 90%, 95%, 96%, 97%, 98%, or 99% sequence homology with a nucleotide sequence of a gRNA described elsewhere herein.

The guide RNA sequence can be a sense or anti-sense sequence. In the CRISPR-Cas system derived from *S. pyogenes,* the target DNA typically immediately precedes a 5'-NGG proto-spacer adjacent motif (PAM). Other Cas9 orthologs may have different PAM specificities. For example, Cas9 from *S. thermophilus* requires 5'-NNAGAA for CRISPR 1 and 5'-NGGNG for CRISPR3) and *Neisseria meningiditis* requires 5'-NNNNGATT). The specific sequence of the guide RNA may vary, but, regardless of the sequence, useful guide RNA sequences will be those that minimize off-target effects while achieving high efficiency mutation of the herpesvirus target sequence(s). The specific sequence of the guide RNA may vary, but, regardless of the sequence, useful guide RNA sequences will be those that minimize off-target effects while achieving high efficiency editing of the HSV genome. The length of the guide RNA sequence can vary from about 20 to about 60 or more nucleotides, for example about 20, about 21, about 22, about 23, about 24, about 25, about 26, about 27, about 28, about 29, about 30, about 31, about 32, about 33, about 34, about 35, about 36, about 37, about 38, about 39, about 40, about 45, about 50, about 55, about 60 or more nucleotides. Useful selection methods identify regions having extremely low homology between the foreign viral genome and host cellular genome, include bioinformatic screening using target sequence+NGG targetselection criteria to exclude off-target human transcriptome or (even rarely) untranslated-genomic sites, and WGS, Sanger sequencing and SURVEYOR assay, to identify and exclude potential off-target effects. Algorithms, such as CRISPR Design Tool (CRISPR Genome Engineering Resources; Broad Institute) can be used to identify target sequences with or near requisite PAM sequences as defined by the type of Cas peptide (i.e. Cas9, Cas9 variant, Cpf1) used.

The composition comprises nucleic acids encoding multiple different gRNA molecules, each targeted to a different target sequence. In certain embodiments, this multiplexed strategy provides for increased efficacy.

In certain embodiments, the RNA molecules (e.g., crRNA, tracrRNA, gRNA) may be engineered to comprise one or more modified nucleobases. For example, known modifications of RNA molecules can be found, for example, in Genes VI, Chapter 9 ("Interpreting the Genetic Code"), Lewis, ed. (1997, Oxford University Press, New York), and Modification and Editing of RNA, Grosjean and Benne, eds. (1998, ASM Press, Washington DC). Modified RNA components include the following: 2'-O-methylcytidine; N⁴-methylcytidine; N⁴-2'-O-dimethylcytidine; N⁴-acetylcytidine; 5-methylcytidine; 5,2'-O-dimethylcytidine; 5-hydroxymethylcytidine; 5-formylcytidine; 2'-O-methyl-5-formaylcytidine; 3-methylcytidine; 2-thiocytidine; lysidine; 2'-O-methyluridine; 2-thiouridine; 2-thio-2'-O-methyluridine; 3,2'-O-dimethyluridine; 3-(3-amino-3-carboxypropyl)uridine; 4-thiouridine; ribosylthymine; 5,2'-O-dimethyluridine; 5-methyl-2-thiouridine; 5-hydroxyuridine; 5-methoxyuridine; uridine 5-oxyacetic acid; uridine 5-oxyacetic acid methyl ester; 5-carboxymethyluridine; 5-methoxycarbonylmethyluridine; 5-methoxycarbonylmethyl-2'-O-methyluridine; 5-methoxycarbonylmethyl-2'-thiouridine; 5-carbamoylmethyluridine; 5-carbamoylmethyl-2'-O-methyluridine; 5-(carboxyhydroxymethyl)uridine; 5-(carboxyhydroxymethyl) uridinemethyl ester; 5-aminomethyl-2-thiouridine; 5-methylaminomethyluridine; 5-methylaminomethyl-2-thiouridine; 5-methylaminomethyl-2-selenouridine; 5-carboxymethylaminomethyluridine; 5-carboxymethylaminomethyl-2'-O-methyluridine; 5-carboxymethylaminomethyl-2-thiouridine; dihydrouridine; dihydroribosylthymine; 2'-methyladenosine; 2-methyladenosine; N⁶N-methyladenosine; N⁶, N⁶-dimethyladenosine; N⁶,2'-O-trimethyladenosine; 2-methylthio-N⁶N-isopentenyladenosine; N⁶-(cis-hydroxyisopentenyl)-adenosine; 2-methylthio-N⁶-(cis-- hydroxyisopentenyl)-adenosine; N⁶-glycinylcarbamoyl)adenosine; N⁶-threonylcarbamoyl adenosine; N⁶-methyl-N⁶-threonylcarbamoyl adenosine; 2-methylthio-N⁶-methyl-N⁶-threonylcarbamoyl adenosine; N⁶-hydroxynorvalylcarbamoyl adenosine; 2-methylthio-N⁶-hydroxnorvalylcarbamoyl adenosine; 2'-O-ribosyladenosine (phosphate); inosine; 2'O-methyl inosine; 1-methyl inosine; 1;2'-O-dimethyl inosine; 2'-O-methyl guanosine; 1-methyl guanosine; N²-methyl guanosine; N²,N²-dimethyl guanosine; N², 2'-O-dimethyl guanosine; N², N², 2'-O-trimethyl guanosine; 2'-O-ribosyl guanosine (phosphate); 7-methyl guanosine; N²;7-dimethyl guanosine; N²; N²;7-trimethyl guanosine; wyosine; methylwyosine; under-modified hydroxywybutosine; wybutosine; hydroxywybutosine; peroxywybutosine; queuosine; epoxyqueuosine; galactosyl-queuosine; mannosyl-queuosine; 7-cyano-7-deazaguanosine; arachaeosine [also called 7-formamido-7-deazaguanosine]; and 7-aminomethyl-7-deazaguanosine. The methods of the present invention or others in the art can be used to identify additional modified RNA molecules.

The isolated nucleic acid molecules of the invention may be produced by standard techniques. For example, polymerase chain reaction (PCR) techniques can be used to obtain an isolated nucleic acid containing a nucleotide sequence described herein, including nucleotide sequences encoding a polypeptide described herein. PCR can be used to amplify specific sequences from DNA as well as RNA, including sequences from total genomic DNA or total cellular RNA. Various PCR methods are described in, for example, PCR Primer: A Laboratory Manual, 2nd edition, Dieffenbach and Dveksler, eds., Cold Spring Harbor Laboratory Press, 2003. Generally, sequence information from the ends of the region of interest or beyond is employed to design oligonucleotide primers that are identical or similar in sequence to opposite strands of the template to be amplified. Various PCR strategies also are available by which site-specific nucleotide sequence modifications can be introduced into a template nucleic acid.

The isolated nucleic acids also can be chemically synthesized, either as a single nucleic acid molecule (*e.g*., using automated DNA synthesis in the 3' to 5' direction using phosphoramidite technology) or as a series of oligonucleotides. Isolated nucleic acids of the invention also can be obtained by mutagenesis of, *e.g.,* a naturally occurring portion crRNA, tracrRNA, RNA-encoding DNA, or of a Cas9 - encoding DNA

### Cas peptide

The composition comprises an isolated nucleic acid encoding a CRISPR-associated (Cas) peptide, or functional fragment or derivative thereof. In certain embodiments, the Cas peptide is an endonuclease, including but not limited to the Cas9 nuclease. In one embodiment, the Cas9 peptide comprises an amino acid sequence identical to the wild type *Streptococcus pyogenes* Cas9 amino acid sequence. In some embodiments, the Cas peptide may comprise the amino acid sequence of a Cas protein from other species, for example other *Streptococcus species,* such as *thermophilus; Psuedomonas aeruginosa, Escherichia coli,* or other sequenced bacteria genomes and archaea, or other prokaryotic microogranisms. Other Cas peptides, useful for the present invention, known or can be identified, using methods known in the art (see e.g., Esvelt et al., 2013, Nature Methods, 10: 1116-1121). In certain embodiments, the Cas peptide may comprise a modified amino acid sequence, as compared to its natural source. For example, in one embodiment, the wild type *Streptococcus pyogenes* Cas9 sequence can be modified. In certain embodiments, the amino acid sequence can be codon optimized for efficient expression in human cells (i.e., "humanized) or in a species of interest. A humanized Cas9 nuclease sequence can be for example, the Cas9 nuclease sequence encoded by any of the expression vectors listed in Genbank accession numbers KM099231.1 GL669193757; KM099232.1 GL669193761; or KM099233.1 GL669193765. Alternatively, the Cas9 nuclease sequence can be for example, the sequence contained within a commercially available vector such as PX330 or PX260 from Addgene (Cambridge, MA). In some embodiments, the Cas9 endonuclease can have an amino acid sequence that is a variant or a fragment of any of the Cas9 endonuclease sequences of Genbank accession numbers KM099231.1 GL669193757; KM099232.1 GL669193761 ; or KM099233.1 GL669193765 or Cas9 amino acid sequence of PX330 or PX260 (Addgene, Cambridge, MA).

The Cas9 nucleotide sequence can be modified to encode biologically active variants of Cas9, and these variants can have or can include, for example, an amino acid sequence that differs from a wild type Cas9 by virtue of containing one or more mutations (e.g. , an addition, deletion, or substitution mutation or a combination of such mutations). One or more of the substitution mutations can be a substitution (e.g., a conservative amino acid substitution).

In certain embodiments, the Cas peptide is a mutant Cas9, wherein the mutant Cas9 reduces the off-target effects, as compared to wild-type Cas9. In one embodiment, the mutant Cas9 is a Streptococcus pyogenes Cas9 (SpCas9) variant.

In one embodiment, SpCas9 variants comprise one or more point mutations, including, but not limited to R780A, K810A, K848A, K855A, H982A, K1003A, and R1060A (Slaymaker et al., 2016, Science, 351(6268): 84-88). In one embodiment, SpCas9 variants comprise D1135E point mutation (Kleinstiver et al., 2015, Nature, 523(7561): 481-485). In one embodiment, SpCas9 variants comprise one or more point mutations, including, but not limited to N497A, R661A, Q695A, Q926A, D1135E, L169A, and Y450A (Kleinstiver et al., 2016, Nature, doi:10.1038/nature16526). In one embodiment, SpCas9 variants comprise one or more point mutations, including but not limited to M495A, M694A, and M698A. Y450 is involved with hydrophobic base pair stacking. N497, R661, Q695, Q926 are involved with residue to base hydrogen bonding contributing to off-target effects. N497 hydrogen bonding through peptide backbone. L169A is involved with hydrophobic base pair stacking. M495A, M694A, and H698A are involved with hydrophobic base pair stacking.

In one embodiment, SpCas9 variants comprise one or more point mutations at one or more of the following residues: R780, K810, K848, K855, H982, K1003, R1060, D1135, N497, R661, Q695, Q926, L169, Y450, M495, M694, and M698. In one embodiment, SpCas9 variants comprise one or more point mutations selected from the group of: R780A, K810A, K848A, K855A, H982A, K1003A, R1060A, D1135E, N497A, R661A, Q695A, Q926A, L169A, Y450A, M495A, M694A, and M698A.

In one embodiment, the SpCas9 variant comprises the point mutations, relative to wildtype SpCas9, of N497A, R661A, Q695A, and Q926A. In one embodiment, the SpCas9 variant comprises the point mutations, relative to wildtype SpCas9, of N497A, R661A, Q695A, Q926A, and D1135E. In one embodiment, the SpCas9 variant comprises the point mutations, relative to wildtype SpCas9, of N497A, R661A, Q695A, Q926A, and L169A. In one embodiment, the SpCas9 variant comprises the point mutations, relative to wildtype SpCas9, of N497A, R661A, Q695A, Q926A, and Y450A. In one embodiment, the SpCas9 variant comprises the point mutations, relative to wildtype SpCas9, of N497A, R661A, Q695A, Q926A, and M495A. In one embodiment, the SpCas9 variant comprises the point mutations, relative to wildtype SpCas9, of N497A, R661A, Q695A, Q926A, and M694A. In one embodiment, the SpCas9 variant comprises the point mutations, relative to wildtype SpCas9, of N497A, R661A, Q695A, Q926A, and H698A. In one embodiment, the SpCas9 variant comprises the point mutations, relative to wildtype SpCas9, of N497A, R661A, Q695A, Q926A, D1135E, and L169A. In one embodiment, the SpCas9 variant comprises the point mutations, relative to wildtype SpCas9, of N497A, R661A, Q695A, Q926A, D1135E, and Y450A. In one embodiment, the SpCas9 variant comprises the point mutations, relative to wildtype SpCas9, of N497A, R661A, Q695A, Q926A, D1135E, and M495A. In one embodiment, the SpCas9 variant comprises the point mutations, relative to wildtype SpCas9, of N497A, R661A, Q695A, Q926A, D1135E, and M694A. In one embodiment, the SpCas9 variant comprises the point mutations, relative to wildtype SpCas9, of N497A, R661A, Q695A, Q926A, D1135E, and M698A.

In one embodiment, the SpCas9 variant comprises the point mutations, relative to wildtype SpCas9, of R661A, Q695A, and Q926A. In one embodiment, the SpCas9 variant comprises the point mutations, relative to wildtype SpCas9, of R661A, Q695A, Q926A, and D1135E. In one embodiment, the SpCas9 variant comprises the point mutations, relative to wildtype SpCas9, of R661A, Q695A, Q926A, and L169A. In one embodiment, the SpCas9 variant comprises the point mutations, relative to wildtype SpCas9, of R661A, Q695A, Q926A, and Y450A. In one embodiment, the SpCas9 variant comprises the point mutations, relative to wildtype SpCas9, of R661A, Q695A, Q926A, and M495A. In one embodiment, the SpCas9 variant comprises the point mutations, relative to wildtype SpCas9, of R661A, Q695A, Q926A, and M694A. In one embodiment, the SpCas9 variant comprises the point mutations, relative to wildtype SpCas9, of R661A, Q695A, Q926A, and H698A. In one embodiment, the SpCas9 variant comprises the point mutations, relative to wildtype SpCas9, of R661A, Q695A, Q926A, D1135E, and L169A. In one embodiment, the SpCas9 variant comprises the point mutations, relative to wildtype SpCas9, of R661A, Q695A, Q926A, D1135E, and Y450A. In one embodiment, the SpCas9 variant comprises the point mutations, relative to wildtype SpCas9, of R661A, Q695A, Q926A, D1135E, and M495A. In one embodiment, the SpCas9 variant comprises the point mutations, relative to wildtype SpCas9, of R661A, Q695A, Q926A, D1135E, and M694A. In one embodiment, the SpCas9 variant comprises the point mutations, relative to wildtype SpCas9, of R661A, Q695A, Q926A, D1135E, and M698A.

In one embodiment, the mutant Cas9 comprises one or more mutations that alter PAM specificity (Kleinstiver et al., 2015, Nature, 523(7561):481-485; Kleinstiver et al., 2015, Nat Biotechnol, 33(12): 1293-1298). In one embodiment, the mutant Cas9 comprises one or more mutations that alter the catalytic activity of Cas9, including but not limited to D10A in RuvC and H840A in HNH (Cong et al., 2013; Science 339 : 919-823, Gasiubas et al., 2012;PNAS 109:E2579-2586 Jinek et al ; 2012; Science 337: 816-821).

However, the present invention is not limited to the use of Cas9-mediated gene editing. Rather, the present invention encompasses the use of other CRISPR-associated peptides, which can be targeted to a targeted sequence using a gRNA and can edit to target site of interest. For example, in one embodiment, the invention utilizes Cpf1 to edit the target site of interest. Cpf1 is a single crRNA-guided, class 2 CRISPR effector protein which can effectively edit target DNA sequences in human cells. Exemplary Cpf1 includes, but is not limited to, *Acidaminococcus sp.* Cpf1 (AsCpf1) and *Lachnospiraceae bacterium* Cpf1 (LbCpf1).

The invention should also be construed to include any form of a peptide having substantial homology to a Cas peptide (e.g., Cas9) disclosed herein. Preferably, a peptide which is "substantially homologous" is about 50% homologous, more preferably about 70% homologous, even more preferably about 80% homologous, more preferably about 90% homologous, even more preferably, about 95% homologous, and even more preferably about 99% homologous to amino acid sequence of a Cas peptide disclosed herein.

The peptide may alternatively be made by recombinant means or by cleavage from a longer polypeptide. The composition of a peptide may be confirmed by amino acid analysis or sequencing.

The variants of the peptides may be (i) one in which one or more of the amino acid residues are substituted with a conserved or non-conserved amino acid residue (preferably a conserved amino acid residue) and such substituted amino acid residue may or may not be one encoded by the genetic code, (ii) one in which there are one or more modified amino acid residues, e.g., residues that are modified by the attachment of substituent groups, (iii) one in which the peptide is an alternative splice variant of the peptide of the present invention, (iv) fragments of the peptides and/or (v) one in which the peptide is fused with another peptide, such as a leader or secretory sequence or a sequence which is employed for purification (for example, His-tag) or for detection (for example, Sv5 epitope tag). The fragments include peptides generated via proteolytic cleavage (including multi-site proteolysis) of an original sequence. Variants may be post-translationally, or chemically modified. Such variants are deemed to be within the scope of those skilled in the art from the teaching herein.

As known in the art the "similarity" between two peptides is determined by comparing the amino acid sequence and its conserved amino acid substitutes of one polypeptide to a sequence of a second polypeptide. Variants are defined to include peptide sequences different from the original sequence, preferably different from the original sequence in less than 40% of residues per segment of interest, more preferably different from the original sequence in less than 25% of residues per segment of interest, more preferably different by less than 10% of residues per segment of interest, most preferably different from the original protein sequence in just a few residues per segment of interest and at the same time sufficiently homologous to the original sequence to preserve the functionality of the original sequence. The present invention includes amino acid sequences that are at least 60%, 65%, 70%, 72%, 74%, 76%, 78%, 80%, 90%, or 95% similar or identical to the original amino acid sequence. The degree of identity between two peptides is determined using computer algorithms and methods that are widely known for the persons skilled in the art. The identity between two amino acid sequences is preferably determined by using the BLASTP algorithm [BLAST Manual, Altschul, S., et al., NCBI NLM NIH Bethesda, Md. 20894, Altschul, S., et al., J. Mol. Biol. 215: 403-410 (1990)].

The peptides can be post-translationally modified. For example, posttranslational modifications that fall within the scope of the present invention include signal peptide cleavage, glycosylation, acetylation, isoprenylation, proteolysis, myristoylation, protein folding and proteolytic processing, etc. Some modifications or processing events require introduction of additional biological machinery. For example, processing events, such as signal peptide cleavage and core glycosylation, are examined by adding canine microsomal membranes or Xenopus egg extracts (U.S. Pat. No. 6,103,489) to a standard translation reaction.

The peptides may include unnatural amino acids formed by posttranslational modification or by introducing unnatural amino acids during translation. A variety of approaches are available for introducing unnatural amino acids during protein translation.

A peptide or protein encoded by the nucleic acids in the composition for the use of the invention may be conjugated with other molecules, such as proteins, to prepare fusion proteins.

A peptide or protein may be phosphorylated using conventional methods such as the method described in Reedijk et al. (The EMBO Journal 11(4):1365, 1992).

Cyclic derivatives of the peptides of the invention are also part of the present invention. Cyclization may allow the peptide to assume a more favorable conformation for association with other molecules. Cyclization may be achieved using techniques known in the art. For example, disulfide bonds may be formed between two appropriately spaced components having free sulfhydryl groups, or an amide bond may be formed between an amino group of one component and a carboxyl group of another component. The components that form the bonds may be side chains of amino acids, non-amino acid components or a combination of the two. In an embodiment of the invention, cyclic peptides may comprise a beta-turn in the right position. Beta-turns may be introduced into the peptides of the invention by adding the amino acids Pro-Gly at the right position.

It may be desirable to produce a cyclic peptide which is more flexible than the cyclic peptides containing peptide bond linkages as described above. A more flexible peptide may be prepared by introducing cysteines at the right and left position of the peptide and forming a disulphide bridge between the two cysteines. The two cysteines are arranged so as not to deform the beta-sheet and turn. The peptide is more flexible as a result of the length of the disulfide linkage and the smaller number of hydrogen bonds in the beta-sheet portion. The relative flexibility of a cyclic peptide can be determined by molecular dynamics simulations.

The invention also envisages peptides comprising a Cas peptide fused to, or integrated into, a target protein, and/or a targeting domain capable of directing the chimeric protein to a desired cellular component or cell type or tissue. The chimeric proteins may also contain additional amino acid sequences or domains. The chimeric proteins are recombinant in the sense that the various components are from different sources, and as such are not found together in nature (i.e. are heterologous).

In one embodiment, the targeting domain can be a membrane spanning domain, a membrane binding domain, or a sequence directing the protein to associate with for example vesicles or with the nucleus. In one embodiment, the targeting domain can target a peptide to a particular cell type or tissue. For example, the targeting domain can be a cell surface ligand or an antibody against cell surface antigens of a target tissue (e.g. cancerous tissue). A targeting domain may target the peptide of the invention to a cellular component. In certain embodiments, the targeting domain targets a tumor-specific antigen or tumor-associated antigen.

N-terminal or C-terminal fusion proteins comprising a peptide or chimeric protein of the invention conjugated with other molecules may be prepared by fusing, through recombinant techniques, the N-terminal or C-terminal of the peptide or chimeric protein, and the sequence of a selected protein or selectable marker with a desired biological function. The resultant fusion proteins contain the Cas peptide or chimeric protein fused to the selected protein or marker protein as described herein. Examples of proteins which may be used to prepare fusion proteins include immunoglobulins, glutathione-S-transferase (GST), hemagglutinin (HA), and truncated myc.

### Nucleic Acids and Vectors

The composition for the use of the invention comprises an isolated nucleic acid encoding one or more elements of the CRISPR-Cas system described herein. In one embodiment, the composition comprises an isolated nucleic acid encoding multiple guide nucleic acid molecules (e.g., gRNA) and encoding a Cas peptide, or functional fragment or derivative thereof.

In one embodiment, the composition comprises at least one isolated nucleic acid encoding multiple gRNAs, where the gRNAs are complementary to target sequences of the HSV genome, as described elsewhere herein. In one embodiment, the composition comprises at least one isolated nucleic acid encoding a gRNA, where the gRNA is complementary to a target sequence having at least 75%, 80%, 85%, 90%, 95%, 96%, 97%, 98%, or 99% sequence homology to a target sequence described herein.

In one embodiment, the composition comprises at least one isolated nucleic acid encoding a Cas peptide described elsewhere herein, or a functional fragment or derivative thereof. In one embodiment, the composition comprises at least one isolated nucleic acid encoding a Cas peptide having at least 75%, 80%, 85%, 90%, 95%, 96%, 97%, 98%, or 99% amino acid sequence homology with a Cas peptide described elsewhere herein.

The isolated nucleic acid may comprise any type of nucleic acid, including, but not limited to DNA and RNA. For example, in one embodiment, the composition comprises an isolated DNA molecule, including for example, an isolated cDNA molecule, encoding a gRNA or peptide of the invention, or functional fragment thereof. In one embodiment, the composition comprises an isolated RNA molecule encoding a peptide of the invention, or a functional fragment thereof. The isolated nucleic acids may be synthesized using any method known in the art.

The present invention also includes a vector in which the isolated nucleic acid of the present invention is inserted. The art is replete with suitable vectors that are useful in the present invention. Vectors include, for example, viral vectors (such as adenoviruses ("Ad"), adeno-associated viruses (AAV), and vesicular stomatitis virus (VSV) and retroviruses), liposomes and other lipid-containing complexes, and other macromolecular complexes capable of mediating delivery of a polynucleotide to a host cell. Vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. Other vectors include those described by Chen et al; BioTechniques, 34: 167-171 (2003). A large variety of such vectors is known in the art and is generally available.

In brief summary, the expression of natural or synthetic nucleic acids encoding an RNA and/or peptide is typically achieved by operably linking a nucleic acid encoding the RNA and/or peptide or portions thereof to a promoter, and incorporating the construct into an expression vector. The vectors to be used are suitable for replication and, optionally, integration in eukaryotic cells. Typical vectors contain transcription and translation terminators, initiation sequences, and promoters useful for regulation of the expression of the desired nucleic acid sequence.

The vectors of the present invention may also be used for nucleic acid immunization and gene therapy, using standard gene delivery protocols. Methods for gene delivery are known in the art. *See, e.g.,* U.S. Pat. Nos. 5,399,346, 5,580,859, 5,589,466, incorporated by reference herein in their entireties. In another embodiment, the invention provides a gene therapy vector.

The isolated nucleic acid of the composition for the use of the invention can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the vector may be provided to a cell in the form of a viral vector. Viral vector technology is well known in the art and is described, for example, in Sambrook et al. (2001, Molecular Cloning: A Laboratory Manual, Cold Spring Harbor Laboratory, New York), and in other virology and molecular biology manuals. Viruses, which are useful as vectors include, but are not limited to, retroviruses, adenoviruses, adeno- associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In one embodiment, lentivirus vectors are used.

For example, vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity. In one embodiment, the composition includes a vector derived from an adeno-associated virus (AAV). Adeno-associated viral (AAV) vectors have become powerful gene delivery tools for the treatment of various disorders. AAV vectors possess a number of features that render them ideally suited for gene therapy, including a lack of pathogenicity, minimal immunogenicity, and the ability to transduce postmitotic cells in a stable and efficient manner. Expression of a particular gene contained within an AAV vector can be specifically targeted to one or more types of cells by choosing the appropriate combination of AAV serotype, promoter, and delivery method.

Pox viral vectors introduce the gene into the cells cytoplasm. Avipox virus vectors result in only a short term expression of the nucleic acid. Adenovirus vectors, adeno-associated virus vectors and herpes simplex virus (HSV) vectors may be an indication for some invention embodiments. The adenovirus vector results in a shorter term expression (e.g., less than about a month) than adeno-associated virus, in some embodiments, may exhibit much longer expression. The particular vector chosen will depend upon the target cell and the condition being treated.

In certain embodiments, the vector also includes conventional control elements which are operably linked to the transgene in a manner which permits its transcription, translation and/or expression in a cell transfected with the plasmid vector or infected with the virus produced by the invention. As used herein, "operably linked" sequences include both expression control sequences that are contiguous with the gene of interest and expression control sequences that act in trans or at a distance to control the gene of interest. Expression control sequences include appropriate transcription initiation, termination, promoter and enhancer sequences; efficient RNA processing signals such as splicing and polyadenylation (polyA) signals; sequences that stabilize cytoplasmic mRNA; sequences that enhance translation efficiency (i.e., Kozak consensus sequence); sequences that enhance protein stability; and when desired, sequences that enhance secretion of the encoded product. A great number of expression control sequences, including promoters which are native, constitutive, inducible and/or tissue-specific, are known in the art and may be utilized.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

The selection of appropriate promoters can readily be accomplished. In certain aspects, one would use a high expression promoter. One example of a suitable promoter is the immediate early cytomegalovirus (CMV) promoter sequence. This promoter sequence is a strong constitutive promoter sequence capable of driving high levels of expression of any polynucleotide sequence operatively linked thereto. The Rous sarcoma virus (RSV) and MMT promoters may also be used. Certain proteins can be expressed using their native promoter. Other elements that can enhance expression can also be included such as an enhancer or a system that results in high levels of expression such as a tat gene and tar element. This cassette can then be inserted into a vector, e.g., a plasmid vector such as, pUC19, pUC118, pBR322, or other known plasmid vectors, that includes, for example, an *E. coli* origin of replication.

Another example of a suitable promoter is Elongation Growth Factor - 1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatinine kinase promoter. Further, the invention should not be limited to the use of constitutive promoters. Inducible promoters are also contemplated as part of the invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

Enhancer sequences found on a vector also regulates expression of the gene contained therein. Typically, enhancers are bound with protein factors to enhance the transcription of a gene. Enhancers may be located upstream or downstream of the gene it regulates. Enhancers may also be tissue-specific to enhance transcription in a specific cell or tissue type. In one embodiment, the vector of the present invention comprises one or more enhancers to boost transcription of the gene present within the vector.

In order to assess the expression of the nucleic acid and/or peptide, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2012, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL). Stock solutions of lipids in chloroform or chloroform/methanol can be stored at about -20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectaminenucleic acid complexes.

Regardless of the method used to introduce exogenous nucleic acids into a host cell, in order to confirm the presence of the recombinant nucleic acid sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the invention.

In certain embodiments, the composition comprises a cell genetically modified to express one or more isolated nucleic acids and/or peptides described herein. For example, the cell may be transfected or transformed with one or more vectors comprising an isolated nucleic acid sequence encoding a gRNA and/or a Cas peptide. The cell can be the subject's cells or they can be haplotype matched or a cell line. The cells can be irradiated to prevent replication. In some embodiments, the cells are human leukocyte antigen (HLA)-matched, autologous, cell lines, or combinations thereof. In other embodiments the cells can be a stem cell. For example, an embryonic stem cell or an artificial pluripotent stem cell (induced pluripotent stem cell (iPS cell)). Embryonic stem cells (ES cells) and artificial pluripotent stem cells (induced pluripotent stem cell, iPS cells) have been established from many animal species, including humans. These types of pluripotent stem cells would be the most useful source of cells for regenerative medicine because these cells are capable of differentiation into almost all of the organs by appropriate induction of their differentiation, with retaining their ability of actively dividing while maintaining their pluripotency. iPS cells, in particular, can be established from self-derived somatic cells, and therefore are not likely to cause ethical and social issues, in comparison with ES cells which are produced by destruction of embryos. Further, iPS cells, which are a self-derived cell, make it possible to avoid rejection reactions, which are the biggest obstacle to regenerative medicine or transplantation therapy.

### Pharmaceutical Compositions

The compositions described herein are suitable for use in a variety of drug delivery systems described above. Additionally, in order to enhance the in vivo serum half-life of the administered compound, the compositions may be encapsulated, introduced into the lumen of liposomes, prepared as a colloid, or other conventional techniques may be employed which provide an extended serum half-life of the compositions. A variety of methods are available for preparing liposomes, as described in, e.g., Szoka, et al., U.S. Pat. Nos. 4,235,871, 4,501,728 and 4,837,028 each of which is incorporated herein by reference. Furthermore, one may administer the drug in a targeted drug delivery system, for example, in a liposome coated with a tissue-specific antibody. The liposomes will be targeted to and taken up selectively by the organ.

The present invention also provides pharmaceutical compositions comprising one or more of the compositions described herein. Formulations may be employed in admixtures with conventional excipients, i.e., pharmaceutically acceptable organic or inorganic carrier substances suitable for administration to the wound or treatment site. The pharmaceutical compositions may be sterilized and if desired mixed with auxiliary agents, e.g., lubricants, preservatives, stabilizers, wetting agents, emulsifiers, salts for influencing osmotic pressure buffers, coloring, and/or aromatic substances and the like. They may also be combined where desired with other active agents, e.g., other analgesic agents.

Administration of the compositions of this invention may be carried out, for example, by parenteral, by intravenous, intratumoral, subcutaneous, intramuscular, or intraperitoneal injection, or by infusion or by any other acceptable systemic method. Formulations for administration of the compositions include those suitable for rectal, nasal, oral, topical (including buccal and sublingual), vaginal or parenteral (including subcutaneous, intramuscular, intravenous and intradermal) administration. The formulations may conveniently be presented in unit dosage form, e.g. tablets and sustained release capsules, and may be prepared by any methods well known in the art of pharmacy.

As used herein, "additional ingredients" include, but are not limited to, one or more of the following: excipients; surface active agents; dispersing agents; inert diluents; granulating and disintegrating agents; binding agents; lubricating agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other "additional ingredients" that may be included in the pharmaceutical compositions of the invention are known in the art and described, for example in Genaro, ed. (1985, Remington's Pharmaceutical Sciences, Mack Publishing Co., Easton, PA), which is incorporated herein by reference.

The composition of the invention may comprise a preservative from about 0.005% to 2.0% by total weight of the composition. The preservative is used to prevent spoilage in the case of exposure to contaminants in the environment. Examples of preservatives useful in accordance with the invention included but are not limited to those selected from the group consisting of benzyl alcohol, sorbic acid, parabens, imidurea and combinations thereof. A particularly preferred preservative is a combination of about 0.5% to 2.0% benzyl alcohol and 0.05% to 0.5% sorbic acid.

In an embodiment, the composition includes an anti-oxidant and a chelating agent that inhibits the degradation of one or more components of the composition. Preferred antioxidants for some compounds are BHT, BHA, alphatocopherol and ascorbic acid in the preferred range of about 0.01% to 0.3% and more preferably BHT in the range of 0.03% to 0.1% by weight by total weight of the composition. Preferably, the chelating agent is present in an amount of from 0.01% to 0.5% by weight by total weight of the composition. Particularly preferred chelating agents include edetate salts (e.g. disodium edetate) and citric acid in the weight range of about 0.01% to 0.20% and more preferably in the range of 0.02% to 0.10% by weight by total weight of the composition. The chelating agent is useful for chelating metal ions in the composition that may be detrimental to the shelf life of the formulation. While BHT and disodium edetate are the particularly preferred antioxidant and chelating agent respectively for some compounds, other suitable and equivalent antioxidants and chelating agents may be substituted therefore as would be known to those skilled in the art.

Liquid suspensions may be prepared using conventional methods to achieve suspension the composition of the invention in an aqueous or oily vehicle. Aqueous vehicles include, for example, water, and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions may further comprise one or more additional ingredients including, but not limited to, suspending agents, dispersing or wetting agents, emulsifying agents, demulcents, preservatives, buffers, salts, flavorings, coloring agents, and sweetening agents. Oily suspensions may further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, and hydroxypropylmethylcellulose. Known dispersing or wetting agents include, but are not limited to, naturally-occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (e.g., polyoxyethylene stearate, heptadecaethyleneoxycetanol, polyoxyethylene sorbitol monooleate, and polyoxyethylene sorbitan monooleate, respectively). Known emulsifying agents include, but are not limited to, lecithin, and acacia. Known preservatives include, but are not limited to, methyl, ethyl, or n-propyl-para- hydroxybenzoates, ascorbic acid, and sorbic acid.

### Methods of treatment

The composition of the present invention are useful in a method of treating or preventing herpesvirus-mediated infection. In one embodiment, the method comprises administering to a subject in need thereof, an effective amount of a composition comprising at least one of a guide nucleic acid molecule and a Cas peptide, or functional fragment or derivative thereof. In one embodiment, the method comprises administering a composition comprising an isolated nucleic acid encoding at least one of: the guide nucleic acid molecule and a Cas peptide, or functional fragment or derivative thereof. In certain embodiments, the method comprises administering a composition described herein to a subject diagnosed with a herpesvirus infection, at risk for developing a herpesvirus infection, a subject with a latent herpesvirus infection, and the like.

In one embodiment, the method is used to treat or prevent a herpesvirus infection, including but not limited to herpes simplex type I (HSV1), herpes simplex virus 2 (HSV2), human herpresvirus-3 (HHV-3; varicella zoster virus (VZV), human herpesvirus-4 (HHV-4; Epstein-Barr virus (EBV)), human herpesvirus-5 (HHV-5; Cytomegalovirus (CMV)), human herpesvirus-6 (HHV-6; roseolovirus), human herpes virus-7 (HHV-7), and human herpesvirus-8 (HHV-8; Karposi's sarcoma-associated herpesvirus (KSHV)).

The methods of the invention are also employed for treatment or prevention of diseases and disorders associated with herpesvirus infections, including, but not limited to, labial herpes, genital herpes, herpetic encephalitis, chickenpox, shingles, Bell's palsy, vestibular neuritis, and herpetic neuralgia.

Subjects to which administration of the pharmaceutical compositions of the invention is contemplated include, but are not limited to, humans and other primates, mammals including commercially relevant mammals such as non-human primates, cattle, pigs, horses, sheep, cats, and dogs. The therapeutic agents may be administered under a metronomic regimen. As used herein, "metronomic" therapy refers to the administration of continuous low-doses of a therapeutic agent.

The compositions can be administered in conjunction with (e.g., before, simultaneously or following) one or more therapies. For example, in certain embodiments, the method comprises administration of a composition of the invention in conjunction with an additional anti-herpetic therapy, including, but not limited to a TK inhibitor, a UL30 inhibitor, acyclovir, foscarnet, cidofovir, and derivatives thereof.

Dosage, toxicity and therapeutic efficacy of the present compositions can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. The Cas9/gRNA compositions that exhibit high therapeutic indices are preferred. While Cas9/gRNA compositions that exhibit toxic side effects may be used, care should be taken to design a delivery system that targets such compositions to the site of affected tissue in order to minimize potential damage to uninfected cells and, thereby, reduce side effects.

The data obtained from the cell culture assays and animal studies can be used in formulating a range of dosage for use in humans. The dosage of such compositions lies preferably within a range of circulating concentrations that include the ED₅₀ with little or no toxicity. The dosage may vary within this range depending upon the dosage form employed and the route of administration utilized. For any composition used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of a composition (i.e., an effective dosage) means an amount sufficient to produce a therapeutically (e.g., clinically) desirable result. The compositions can be administered from one or more times per day to one or more times per week; including once every other day. The skilled artisan will appreciate that certain factors can influence the dosage and timing required to effectively treat a subject, including but not limited to the severity of the disease or disorder, previous treatments, the general health and/or age of the subject, and other diseases present. Moreover, treatment of a subject with a therapeutically effective amount of the compositions of the invention can include a single treatment or a series of treatments.

The gRNA expression cassette can be delivered to a subject by methods known in the art. In some aspects, the Cas may be a fragment wherein the active domains of the Cas molecule are included, thereby cutting down on the size of the molecule. Thus, the, Cas/gRNA molecules can be used clinically, similar to the approaches taken by current gene therapy.

In one embodiment, the method comprises genetically modifying a cell to express a guide nucleic acid molecule and/or Cas peptide. For example, in one embodiment, the method comprises contacting a cell with an isolated nucleic acid encoding the guide nucleic acid and/or Cas peptide.

In one embodiment, the cell is genetically modified in vivo in the subject in whom the therapy is intended. In certain aspects, for in vivo, delivery the nucleic acid is injected directly into the subject. For example, in one embodiment, the nucleic acid is delivered at the site where the composition is required. In vivo nucleic acid transfer techniques include, but is not limited to, transfection with viral vectors such as adenovirus, Herpes simplex I virus, adeno-associated virus), lipid-based systems (useful lipids for lipid-mediated transfer of the gene are DOTMA, DOPE and DC-Chol, for example), naked DNA, and transposon-based expression systems. Exemplary gene therapy protocols see Anderson et al., Science 256:808-813 (1992). See also WO 93/25673 and the references cited therein. In certain embodiments, the method comprises administering of RNA, for example mRNA, directly into the subject (see for example, Zangi et al., 2013 Nature Biotechnology, 31: 898-907).

For ex vivo treatment, an isolated cell is modified in an ex vivo or in vitro environment. In one embodiment, the cell is autologous to a subject to whom the therapy is intended. Alternatively, the cell can be allogeneic, syngeneic, or xenogeneic with respect to the subject. The modified cells may then be administered to the subject directly.

One skilled in the art recognizes that different methods of delivery may be utilized to administer an isolated nucleic acid into a cell. Examples include: (1) methods utilizing physical means, such as electroporation (electricity), a gene gun (physical force) or applying large volumes of a liquid (pressure); and (2) methods wherein the nucleic acid or vector is complexed to another entity, such as a liposome, aggregated protein or transporter molecule.

The amount of vector to be added per cell will likely vary with the length and stability of the therapeutic gene inserted in the vector, as well as also the nature of the sequence, and is particularly a parameter which needs to be determined empirically, and can be altered due to factors not inherent to the methods of the present invention (for instance, the cost associated with synthesis). One skilled in the art can easily make any necessary adjustments in accordance with the exigencies of the particular situation.

Genetically modified cells may also contain a suicide gene i.e., a gene which encodes a product that can be used to destroy the cell. In many gene therapy situations, it is desirable to be able to express a gene for therapeutic purposes in a host, cell but also to have the capacity to destroy the host cell at will. The therapeutic agent can be linked to a suicide gene, whose expression is not activated in the absence of an activator compound. When death of the cell in which both the agent and the suicide gene have been introduced is desired, the activator compound is administered to the cell thereby activating expression of the suicide gene and killing the cell. Examples of suicide gene/prodrug combinations which may be used are herpes simplex virus-thymidine kinase (HSV-tk) and ganciclovir, acyclovir; oxidoreductase and cycloheximide; cytosine deaminase and 5-fluorocytosine; thymidine kinase thymidilate kinase (Tdk::Tmk) and AZT; and deoxycytidine kinase and cytosine arabinoside.

### EXPERIMENTAL EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the present invention and practice the claimed methods. The following working examples therefore, specifically point out the preferred embodiments of the present invention, and are not to be construed as limiting in any way the remainder of the disclosure.

### Example 1: Cas9-gRNA is capable of cleaving HSV1 DNA.

Experiments were conducted to examine whether a CRISPR system can be utilized to cleave HSV1 DNA. The methods and results are presented herein.

### Design of Cas9/ICP0 gRNA constructs.

The ICPO gene of the HSV1 genome was selected as an initial target due to its essential role in lower (more natural) MOI lytic infection, its key role in the early steps of reactivation from HSV1 latency, and the multiple assays available for its function. Five different gRNAs were initially selected and were tested for conserved targets within exons 1-3 of the ICPO sequence (Figure 3). Oligo pairs corresponding to each gRNA sequence in forward and reverse positions were annealed and then amplified using PCR, cut appropriately and inserted into either the pX458 or pX260 Cas9 vector (Addgene plasmids 48138 & 42229).

### Cas9/ICP0 gRNA constructs cleave ICPO sequences.

Vero cells stably containing ICPO DNA (L7 or F06) were cotransfected with the Cas9/anti-ICP0 gRNA constructs (Figure 3). Genomic DNA from colonies of F06 cells that were stably transfected with anti-Cas9 gRNAs showed was collected and analyzed by surveyor assay for In/Dels caused by the constructs. Shown in Figure 4 is results of a surveyor assay on the F06 cells stably transfected with Cas9/anti-HSV1 ICPO gRNA 2A (abbreviated Cas9/2A) shows a 180 base pair genomic DNA fragment is generated (Figure 4).

### Use of multiple Cas9/ anti-ICPO gRNA constructs specifically removes larger pieces of HSV1 genomic DNA.

Tandem use of Cas9/ICP0 gRNA constructs to clip out fragments of HSV1 genomic DNA is described. When utilized in tandem in transiently transfected cells, two of the anti-HSV1 ICPO gRNAs (2A and 2C) that were developed were able to delete 335 base pair (bp) leaving a 217 bp segment of the ICPO sequence. This deletion leads to truncation of the ICPO protein with an early termination codon (Figure 5). Shown at the top of Figure 5 is a graphic representation of the ICPO exon 2 sequence, including the specific target DNA (tDNA) targeted by anti-ICPO gRNAs 2A and 2C (Figure 5, top). Immediately beneath this sequence is the cloned and sequenced HSV1 DNA of 10 clones of L7 cells that were transiently transfected with Cas9/anti-ICP0 gRNA 2A and Cas9/anti-ICP0 gRNA 2C. DNA cleavage mediated by these two constructs leads to deletion of a 335 base pair segment of the HSV1 ICPO DNA. Eight out of the 10 sequenced clones showed a single base pair deletion (shown as a gap in the DNA alignments below the schematized wild type ICPO sequence. Two of the 10 sequenced clones showed a single base pair insertion at the site (Figure 5, lower right). The Figure 5 inset (lower left) shows an agarose gel demonstrating the excised 217 base pair fragment of ICPO (Figure 5, lower left). Thus, as described earlier, the CRISP-Cas9 constructs developed herein can cleave two gRNA-directed regions within the viral genome and the remaining gene sequence is re-joined by nonhomologous end joining (NHEJ).

### Cas9-gRNA targets HSV1 ICP0 function and suppresses HSV1 infection in cells.

Normally, HSV1 can infect the permissive Vero cell line at a relatively low concentration of virus (Figure 6, first column on the left side of the graph, marked wt Vero wt). Infection of the same Vero cell line with mutant HSV1 virus lacking the ICPO gene requires at least 100x the amount of mutant virus to infect these cells (Figure 6, second column from the left, marked wt Vero ΔICP0). When ICPO is supplemented into the cell line (for example, in F06 cells which express ICPO), then those cells can be infected with ICPO lacking HSV1 at significantly lower concentrations as expected (Figure 6, fourth column from the left on graph, marked F06 ΔICP0). After stable transfection of normal Vero cells with Cas9/anti-HSV1 ICPO gRNA 2D, the cells become significantly more resistant to infection with wildtype HSV1, and cannot be infected with low concentrations of the virus (Figure 6, fifth column on the graph, marked Vero Cas9/2D). These results are equivalent to those seen when it is attempted to infect normal Vero cells with mutant HSV1 virus that completely lacks ICPO (Figure 6, second lane on the graph, marked wt Vero ΔICP0).

The results shown in Figure 6 demonstrated that expression of anti-ICPO gRNA drastically suppressed viral replication, as evidenced by the need for increased amounts of virus to infect Vero cells transfected with Cas9/ anti-HSV1 ICPO gRNA 2D. Taken together, these observations underscore the capabilities of the presently described invention, a novel gene editing system that can destroy the integrity of HSV1 DNA.

### Example 2: Ablation of HSV-1 Replication by Gene Editing Strategy

Herpes simplex virus type 1 (HSV-1) is a ubiquitous and highly contagious human neurotropic virus affecting over 85% of adults worldwide. After primary infection, HSV-1 persists in a latent state within nervous system ganglia, where it can sporadically become reactivated, and replicate after axonal transport to skin, causing painful skin or mucosal lesions. HSV-1 penetration into cerebral parenchyma and replication within frontal and temporal lobes can cause fatal encephalitis. Survivors suffer serious neurological complications permanently, including seizures, motor disorders and mental health deficits. No curative therapy exists for HSV-1 induced illness, and the persistence of reactivatable virus keeps infected individuals at risk for its complications.

Studies of protective responses of single-cell organisms including *Streptococcus pyogenes* against invasion by bacteriophage and transposable DNA elements led to discovery of the CRISPR/Cas9 system (Garneau et al., 2010, Nature, 468, 67-71). The endogenous type II CRISPR system in such microbes apparently defends against foreign DNA, as association of CRISPR RNA (crRNA) and transactivated RNA (trRNA) sequences can trigger targeting of foreign DNA sequences for double-strand cleavage by the DNA endonuclease Cas9. The specificity of such cleavage is determined by complementary base pairing between crRNA and sequences on the target DNA (Gasiunas et al., 2014, Proc Natl Acad Sci USA, 109: 15539-15540). Exploiting this, the Cas9 system has recently been modified to permit specific genomic editing in mammalian cells. Upon entering the nuclei of eukaryotic cells and utilizing the guide RNAs (gRNAs), Cas9 introduces insertion/deletion (InDel) mutations into target genes (Mali et al., 2013, Science, 339: 823-826). CRISPR/Cas9 has been used to modify several eukaryotic genes, and several groups have explored its antiviral potential (Cong et al., 2013, Science, 339: 819-823; Ebina et al., 2013, Sci Rep, 3: 2150; Hu et al., 2014, Proc Natl Acad Sci USA, 111: 11464-11466; Mali et al., 2013, Science, 339: 823-826; Wang et al., 2014, Proc Natal Acad Sci USA, 111: 13157-13162; Wollebo et al., 2011, J Neuroimmunol, 223: 46-53; Yuen et al., 2015, J Gen Virol, 96: 626-636). To assess its translational potential for application to treat and eliminate HSV-1 infection, experiments presented herein were used to examine the ability of CRISPR/Cas9 to suppress HSV-1 replication by targeting specific DNA sequences essential to viral protein expression during early and late phases of viral infection/reactivation.

During primary HSV-1 infection, a defined class of sequentiallyexpressed viral proteins direct a successful productive infection cycle. After viral entry into cells, viral capsid proteins are released within the cytoplasm, causing shutdown of host protein synthesis. Also during this immediate early phase of viral infection, 100-200 copies of infected cell protein 0 (ICPO), which is associated with capsid proteins, is transported to the inner tegument and facilitates nuclease-mediated entry of capsid proteins to the sites where viral replication occurs. Concurrently, the viral gene encoding ICPO is activated, and newly-synthesized ICPO promotes efficient progression of lytic infection, which is more similar to real-life infection than high dose infection models. Therefore, ICPO contributes more effectively to viral survival and replication during low-dose viral infection. ICPO is also important in HSV-1 reactivation and has critical roles in lytic infection, including disruption of PML bodies and avoidance of innate immune responses (Boutell and Everett, 2013, J Gen Virol, 94: 465-481; Samaniego et al, 1997, J Virol, 71: 4614-4625; Hagglund and Roizman, 2004, J Virol, 78: 2169-2178). Therefore, experiments were conducted by targeting DNA sequences of the ICPO gene and by developing guide RNAs to combine with Cas9, to test their ability to suppress viral gene expression. It was found that this strategy introduced specific InDel mutations in the ICPO sequence, produced no off-target effects or toxicity to host cells, and rescued antiviral PML bodies from disintegration by ICPO, supporting the promise of this model approach for developing anti-HSV-1 therapies capable of eliminating latent infection.

To test feasibility of ablating latent HSV from host cells, RNA-guided CRISPR/Cas9 gene editing was used to specifically target for deletion DNA sequences of the HSV-1 genome that span the region directing expression of ICPO, a key viral protein that stimulates HSV-1 gene expression and replication. It was found that CRISPR/Cas9 introduced InDel mutations into exon 2 of the ICPO gene, reducing the ability of ICPO to stimulate HSV-1 infectivity in permissive human cell culture models. Further, co-expression of Cas9 and ICPO-targeting guide RNAs protected permissive cells against HSV-1 infection. Persistent co-expression of Cas9 and gRNA 2A prevented HSV-1-induced disintegration of promonocytic leukemia (PML) nuclear bodies, an intracellular event critical to productive HSV-1 infection that is initiated by interaction of the ICPO N-terminus with PML. Results of SURVEYOR assay excluded any off-target effects that may have introduced mutations in host genes having DNA sequences bioinformatically similar, but not identical, to ICPO. Also, no CRISPR/Cas9-induced apoptosis or adverse side effects upon host cell viability or cell cycle progression was observed, supporting the specificity and prospective safety of this HSV-1 gene-editing strategy. Thus, the data presented herein demonstrate that RNA-guided CRISPR/Cas9 can be used to develop a novel, specific and efficacious therapeutic and prophylactic platform for targeted viral genomic ablation to treat HSV-1 diseases.

The materials and methods employed in these experiments are now described.

### Cell Culture.

ICPO-complementing cell line L7 (Samaniego et al, 1997, J Virol, 71: 4614-4625) was grown in Dulbecco's Modified Eagle's Medium (DMEM) (Life Technologies, NY) supplemented with 10% fetal bovine serum (FBS), 2 mM glutamine and 400 µg/ml of Geneticin (Life Technologies, NY). Normal Vero cells were grown in the same medium without Geneticin. The human oligodendroglioma cell line TC620 were maintained in DMEM supplemented with 10% FBS as previously described (Wollebo et al., 2015, PLoS One, 10, e0136046). For selection, cells were transfected with appropriate vectors and selected using the above medium containing 3 µg of puromycin (Life technologies) for 5 days when they were replated onto 96 cell well plates at 0.5 cell per well in the presence of puromycin. In order to produce clonal derivatives of TC620 cells expressing Cas9 and gRNAs, this cells were transfected with pX260 or pX260-derived plasmids expressing each of the gRNA that are described elsewhere herein. Selection was done with 3 µg/ml puromycin and clones isolated by dilution cloning. All transfections were performed using Lipofectamine 2000 (Life technologies) according to the manufacturer protocol.

Cell viability assay was performed using Live/Death cell viability assay kit (Molecular probes, NY) according to the manufacturer protocol on Vero or L7 cells transfected with different anti-ICPO gRNAs after three days.

### Antibodies.

The following antibodies were used for the Western blot. Mouse α-ICPO (1:1000, Santa Cruz, TX), Mouse monoclonal [11E2] ICP8 (1:1000, Abcam), Mouse monoclonal [3G9] α-gC (1:1000, Abcam) Mouse anti-Flag M2 (1:1000, Sigma Aldrich), anti-αtubulin clone B512 from (1:5000, Sigma Aldrich )

### Apoptosis Assay.

Apoptosis assay was performed according to manufacturer's recommendation (Guava Technologies). Briefly, the control and Cas9 stable expressing cells were plated in 6 well plates at density of 200,000 cells/ml for 48 hrs. Before harvesting the cells, the supernatant of each sample is collected in 15 ml falcon tube and the cells were washed with PBS and trypsinized with 0.25% trypsin and then trypsin was inactivated with the supernatant collected above for each sample. The samples were centrifuged for 5 min by 2000 rpm. The supernatant was aspirated and the pellet was washed with PBS once and the cells were resuspended and counted and diluted to density of 100,000 cells/ml in PBS. 100 µl of room temperature Annexin V-PE staining reagent was mixed with 100 µl of cell suspension and incubated for 25 min at room temperature in the dark. Samples were analyzed using a Guava Easy Cyte mini flow Cytometer.

### Cell cycle analysis.

Control and cas9 stably expressing cells were plated at density of 150,000cells/ml for 48 hrs. Cells were collected and harvested by centrifugation. The pellet was washed once with PBS and resuspended in 1 ml PBS and fixed in ice cold ethanol (70% final concentration). The cells were incubated for 24 hours at -20°C. The cells were centrifuged and the pellet was washed once with PBS containing 1% BSA and stained with Propidium iodide (10 µg/ml) in PBS containing 250 µg/ml RNase A and incubated at 37°C for 45 min in the dark. Cell cycle analysis was performed with the Guava Easy Cyte mini system using the Guavasoft cell cycle program.

### Viability assay.

For viability assay, the control and Cas9 stably expressing cells were plated in triplicate in 96 well plates at a density of 12000 cells/200 µl for 24 hrs. Two hours before the MTT assay, the old media was removed and replaced with the new one. The cells were treated with 20 µl of MTT solution (5 mg/ml MTT in PBS) for 2 hours in 37 °C. After 2 hours, the media was removed and replaced with 200 µl of MTT solvent (4 mM HCl, 0.1% Nondet P-40 (NP40) in isopropanol) and the cells were put on a shaker for 10 min. MTT activity read out was done at channels 590 nm and 620 nm.

### HSV-1 ICPO gRNAs.

The genomic sequence of HSV-1 *ICP0* (NC_0018061) was obtained from NCBI database and *ICP0* exon 2 open reading frame was determined using the published ICPO protein sequence and NCBI database. Three gRNAs were designed and selected using available online tools (http://crispr.mit.edu/). CRISPR Cas9 off target finding tools were used to determine off target sequences.

A pair of DNA oligos from each target sequence were designed in forward and reverse orientations based on published and recommended flanking sequences for PX458 and PX260 vectors (Addgene plasmids 48138 and 42229, respectively). Each pair were annealed in a thermocycler, using 5 µl of each oligo at the concentration of 100 nM at 95°C for 7mins and ramped at 3% from 95°C to 25°C in the presence of 2 µl of T4 DNA ligase buffer and 9 µl of water in 20 µl of total reaction. Annealed oligo pairs were then cloned into BbsI linearized PX458 and PX260 vectors. The insertion of the gRNAs were confirmed using sequencing. Plasmid preps were prepared using Plasmid Midi kit (Qiagen).

### InDel mutation analysis.

Deletions and/or insertions of nucleotides in exon 2 of HSV-1 *ICP0* were verified by single or co-transfection experiments in L7 cells. Two micrograms of PX260 (carrying puromycin resistant gene and ICPO gRNA 2A or 2B) and PX458 constructs (carrying EGFP-Cas9 and ICPO gRNA 2C to estimate the transfection efficiency) were cotransfected for overnight. L7 cells were transfected with empty vectors were served as control. The transfection efficiency was estimated using an inverted fluorescence microscope by examining multiple fields and counting GFP positive cells versus total cell number. Genomic DNA extractions were performed using Archive PureDNA extraction kit (5Prime, MD) after three days. Genomic DNA amplifications were performed using Q5 Hot Start High-Fidelity DNA polymerase (NEB, MA) and two primers which were designed from *ICP0* sequence to amplify a fragment with 552 bp or 470 bp in size (shown in Table 1) using following conditions; 98°C (30s) then 35 cycles with 98°C (10s), 62°C (30s), 72°C (30s) and then 72°C (5 min). The PCR products were analyzed on a 3% agarose gel. The bands of interest were gel-purified and cloned into pCRII T-A vector (Life technologies), and the nucleotide sequence of individual clones was determined by sequencing at Genewiz.

### RT-PCR.

To determine the expression of gRNA 2A in L7 and TC620 stable cells lines, RT-PCR was performed essentially as described previously (Shekarabi et al., 2008, J Clin Invest, 118: 2496-2505), with the exception of using a PX260 based reverse primer (PX260-crRNA-3', Table 1) as a primer to generate the cDNAs. To amplify crRNA, the same primer was paired with the gRNA 2A forward oligo.

### ICP0⁵⁵⁸-Ds-Red Construct.

To generate ICP0⁵⁵⁸-Ds-Red, two primers were designed with Age I restriction sites on each ends and used to amplify a 558 bp fragment from the exon 2 of HSV-1 ICPO genome using Q5 Hot Start (NEB) using ICP0-AgeI-F (3086-3103, NC_0018061) and ICP0-AgeI-R (3626-3643, NC_0018061) (shown in Table 1). pDs-Red-C1 and the amplified fragment were digested with Age I and ligated using T4 DNA ligase (NEB) resulting in a final in-framed with the Ds-Red protein sequence. After selection, the cells were plated onto 96 well plates at the density of 0.5 cells/well to increase the probability of one cell per well seeding. The cells were then allowed to grow to 60%-70% confluence when they were cotransfected with EGFP and ICP0⁵⁵⁸-Ds-Red constructs using Lipofectamine 2000. 48 hours post transfection the cells were examined under an inverted fluorescence microscope and wells with no red signals were selected for further analysis. Genomic DNA were prepared and PCR-amplified using P3 and P4 primers spanning a 372 bp fragment consisting of 2A target sequence (Table 1). The amplified fragments were gel purified using Qiaex II gel extraction kit (Qiagen) and sequenced to verify InDel sequence.

**Table 1: Primers**

| Primer | Sequence |
|---|---|
| P1 | 5'-GATGTCTGGGTGTTTCCCT-3' (SEQ ID NO: 17) |
| P2 | 5'-CACGATCGGGATGGTGCTGAACGACC-3' (SEQ ID NO: 18) |
| P3 | 5'- TGCTGATTGACGCGGGAAATC-3' (SEQ ID NO: 19) |
| P4 | 5'- CGATCTCATCCGTGCACACG-3' (SEQ ID NO: 20) |
| P5 | 5'-CCATCCACGCCCTGCGGCCCCAGC-3'(SEQ ID NO: 21) |

**Table 2: Primers (cellular genes)**

| Primer | Sequence |
|---|---|
| Off 1(G32) | 5'-CACGTCTGTGGGTGTTCAGA-3' (SEQ ID NO: 22) |
| | 5'-AAAGCTCCTAGGGCTGTTGG-3 (SEQ ID NO: 23) |
| Off2 (G42) | 5'-GGAACCCCCTCGCTTTCTTT-3' (SEQ ID NO: 24) |
| | 5'-CGTATGTGTCCCATCGGCAT-3' (SEQ ID NO: 25) |
| off3 (G22) | 5'-CACTGCCTTGTGCCCATAGA-3' (SEQ ID NO: 26) |
| | 5'-TTGAAGCTGCACGTGTTGGA-3' (SEQ ID NO: 27) |
| Off4 (G12) | 5'-ACTGACGGGATCTGGGATCA-3' (SEQ ID NO: 28) |
| | 5'-TTCACTGGGAGACGTCAAGC-3' (SEQ ID NO: 29) |
| off5 (G6) | 5'-GTGAGGCCCCACACATGATT-3' (SEQ ID NO: 30) |
| | 5'-AAGGATGAGAGATGGGGAAGC-3' (SEQ ID NO: 31) |

**Table 3: Sequence of gRNAs**

| Primer | Sequence |
|---|---|
| ICPO 2A | 5'-TCTGGGTGTTTCCCTGCGACCGAGACCTGC-3'(SEQ ID NO: 1) |
| ICPO 2B | 5'-GCATCCCGTGCATGAAAACCTGG-3' (SEQ ID NO: 11) |
| ICPO 2C | 5'- GCATCCCGTGCATGAAAACC-3' (SEQ ID NO: 3) |
| ICPO 2D | 5'-TGTGCAACGCCAAGCTGGTGTACCTGATAG-3' (SEQ ID NO: 4) |
| ICPO 1 | 5'-GCGAGTACCCGCCGGCCTGA-3' (SEQ ID NO: 5) |
| ICPO 3 | 5-GCGAGCCGCGGCGCCGCGGG-3' (SEQ ID NO: 6) |
| ICP4 m1 | 5'-TTCTACGCGCGCTATCGCGACGG-3' (SEQ ID NO: 7) |
| ICP27m 1 | 5'-GGAGTGTTCCTCGTCGGACGAGG-3'(SEQ ID NO: 8) |

### Immunocytochemistry and Western blots.

Two L7 clones were picked and plated onto 8 well chamber slides at 6000 cells per well. They were then infected with 5 MOI of HSV-1 (Patton strain) GFP-Us11 for 2 hours. At 6 hours post infection, the cells were quickly rinsed with cold Balanced Saline Solution (HBSS, Invitrogen) before they were fixed with 4% paraformaldehyde. The cells were immunostained essentially as described elsewhere (Shekarabi and Kennedy, 2002, Mol Cell Neurosci, 19: 1-17). Mouse α-ICP0 (0.4 µg/mL, Santa Cruz, TX), and mouse anti-PML C7 (2 µg/mL, Abcam, MA) antibodies were used for immunostaining at 4 °C for overnight. Alexa Fluor 555 secondary anti-mouse antibody (Molecular Probes; Invitrogen), were used (1: 1,000) to visualize mouse primary antibodies. The nuclei were labeled with 0.5 µg/ml Hoechst 33258 (Sigma, MO) in PBS for 30 min. Imaging was carried out using a Leica TCS SP5 broadband confocal microscope equipped with the AOBS (acousto-optical beam splitter) for optimal beam splitting.

For protein analysis, wells of transiently transfected Vero cells or Puromycin selected L7 clones were infected with 1 MOI of HSV-1 in different times points after infection and washed twice with cold HBSS and protein samples collected with lysis buffer (8M urea, 0.5% SDS, 200mM β-mercaptoethanol). Total protein samples (20 µg) from each condition were run on 8% acrylamide SDS-PAGE gels. Gels were transferred onto nitrocellulose membranes (LI-COR Biosciences; Lincoln, NE) and were blocked 2 hours at room temperature (RT) with 2.5% non-fat dry milk (LabScientific; Highlands, NJ) in PBS-Tween (0.1%, Amresco, OH). Membranes were hybridized with mouse α-ICP0, α-ICP8, α-glycoprotein C or α-FLAG epitope antibody (1µg/mL) at 4°C for overnight. Equivalent loading was determined using mouse α-tubulin antibody (1 µg/mL: Abcam; Cambridge, MA). Membranes were then hybridized with α-mouse or α-rabbit secondary antibodies (both at 1:8000, LI-COR) for 1 hour at room temperature. Membranes were scanned with Odyssey CLX (LI-COR). Images were processed using Image Studio version 2.0 (LI-COR).

For protein analysis of TC620 cells, whole cell extracts were prepared in TNN buffer containing mammalian protease inhibitor cocktail (sigma Aldrich). 50 µg of protein was separated by 10% SDS-PAGE, transferred to nitrocellulose. Blots were blocked in 5% milk in 1XPBST and immunoblotted with primary antibodies (1:1000) for 2 hours at room temperature. After washing, the blots were incubated with secondary antibody Goat Anti-Mouse (1:5000) LI-cor dyes and visualized with an Odyssey CLx Imaging system (LI-COR, INC, Lincoln, NE) using LI-COR Odyssey software.

### Viral titer.

To assay the infection efficiency HSV-1 on gRNA 2A clonal cells, the mock transfected L7 cells or G9 or H5 clones were first infected with one MOI of wild type or ICPO mutant HSV-1 strain (7134, Cai and Schaffer, 1992, J Virol, 66: 2904-2915) for two hours at 37°C in MEM medium (Life Technologies). The supernatant containing newly generated viral particles were then collected and titered by infecting normal Vero cells using serial dilutions. After 24 or 48 hours cells were fixed in 10% Trichloroacetic acid (Sigma) for 10 minutes and stained with 0.05% of Crystal violet (Sigma). The plaques were counted and graphed.

### Infection of TC620 cells with HSV-1.

One day before infection, 45,000 TC620 cells per well were platted in 48 wells plate with DMEM supplemented with 10% FBS. On the day of infection, old media from TC620 cells plated on the day before was aspirated, and 100 µL of diluted virus media was added into each well. After 2 hours of incubation at 37 °C, media containing virus was aspirated, and fresh TC620 media was added back on. Finally, plates were kept at 37 °C for 2 days.

### Production of lentiviral vectors for gRNAs and transduction of TC620 cells stably expressing Cas9.

To construct the gRNA lentiviral expression plasmids for each of the two targets, the U6 expression cassette from each of the two pX330 gRNA plasmids that were described above was amplified by PCR using primers (5'-TATGGGCCCACGCGTGAGGGCCTATTTCCCATGATTCC-3' (SEQ ID NO: 32) and 5'-TGTGGATCCTCGAGGCGGGCCATTTACCGTAAGTTATG-3'(SEQ ID NO: 33)).

The PCR products were cut with MluI and BamH1 and then cloned into pKLV-U6gRNA (BbsI)-PGKpuro2ABFP that had been cut with MluI and BamH1. The pCR^{™}4-TOPO^{®} TA vector was from Life Technologies, Inc., (Carlsbad, CA). To produce lentiviral vectors for transduction of the two gRNAs, each of the two gRNA lentiviral expression plasmid derivatives constructed as described above from pKLV-U6gRNA(BbsI)-PGKpuro2ABFP were transfected into 293T cells by calcium chloride precipitation together with packaging plasmids pCMV-VSV-G, pMDLg/pRRE and pRSV-Rev. Lentivirus was harvested from the supernatant after 48h, cleared by centrifugation and passage through a 0.45 µm filter and added to TC620 cells stably expressing Cas9 in the presence of 6 µg/ml polybrene followed by selection. After 48 hours the cells were harvested and analyzed for ICPO expression by Western blot and viral titer by plaque assay.

### SURVEYOR assay.

The presence of mutations in PCR products of off-target genes derived from TC620 cells stably expressing Cas9 and transduced by lentiviral vectors for gRNAs was examined using the SURVEYOR Mutation Detection Kit (Transgenomic) according to the Manufacturer's protocol. Heterogeneous PCR product was denatured for 10 min at 95°C and then hybridized by gradual cooling using a thermocycler. Three hundred nanograms of hybridized DNA (9 µl) was digested with 0.25 µl of SURVEYOR Nuclease, which is a mismatch-specific DNA endonuclease used to scan for mutations in heteroduplex DNA, plus 0.25 µl SURVEYOR Enhancer S and 15 mM MgCl2 for 4 h at 42 °C. Stop Solution was added and samples were resolved on a 2% agarose gel together with equal amounts of control samples treated in parallel but derived from TC620 cells stably expressing Cas9 but not transduced by lentiviral vector for gRNA.

The results of the experiments are now described.

### gRNA Targeting, Construction and testing in Vero cells

Bioinformatic screening of the HSV-1 genome identified three specific regions within exon II of the ICPO gene, spanning nucleotide (nt) sequences 1011-1040 (2A), 1127-1156 (2B) and 1136-1375 (2C), for creating guide RNAs (gRNAs) for editing by CRISPR/Cas9 (Figure 7A).

Small DNA fragments corresponding to each motif were first cloned separately in expression vector PX260 (Cong et al., 2013, Science, 339: 819-823), and their abilities to induce InDel mutations, when expressed in single form, or to excise a DNA fragment spanning between the cleavage sites, when used in combination, were assessed by PCR amplification and Sanger sequencing. Initially an ICPO-complementing L7 cell line (Samaniego et al, 1997, J Virol, 71: 4614-4625), herein called L7, was used, which contained an integrated copy of the ICPO gene. The cells expressed low levels of the encoded protein when the cells are infected with HSV-1 for transfection with Cas9 along with gRNA 2A- and 2C-expressing plasmids. In addition to an expected 552 bp amplicon, a smaller DNA fragment of 217 bp was also detected in cells expressing both gRNAs 2A and 2C (Figure 7B). Excision of a 335 bp DNA fragment spanning between the 2A and 2C sites was verified by Sanger sequencing, and amplification of a smaller (217 bp) DNA fragment was verified using a template created by re-joining the remaining DNA of exon II.

Further analyses in clonal cell lines showed that in a small population of cells (8%), removal of a 217 bp fragment was accompanied by insertion of a single A or G nucleotide at the cleavage and rejoining sites (Figure 11A). Similarly, multiplex expression of gRNA 2B and 2C caused excision of a 219-bp DNA fragment between 2B and 2C, and amplification of a 251-bp DNA fragment (Figure 11B). In single-gRNA expression configuration, expressing gRNA 2A produced several InDel mutations, detected by Cel1 nuclease-based heteroduplex-specific SURVEYOR assay (Figure 11C). Characterization of the InDel mutations by DNA sequencing of several clonal cells revealed insertion of single or multiple single nucleotides, as well as insertion of a large 191-bp DNA clone close to the cleavage sites (Figure 7D and Figure 7E). As noted, each of these mutations caused a frameshift in the ICPO DNA coding sequences (Figure 7E). The expression of ICPO in control L7 cells was compared to that in clone InDel 3 upon infection with HSV-1 harboring a reporter GFP gene. As shown in Figure 7F (lower panels), infection of control cells with HSV-1/GFP induced nuclear accumulation of ICPO, where newly replicated virus is present. In contrast, Cas9/gRNA 2A prevented detectable ICPO appearance within the nuclei of infected cells, indicating that the frameshift mutations introduced in exon II of the ICPO completely blocked expression of the protein and its subcellular localization (Figure 7F, upper panels). ICPO (red) was co-localized in the cytoplasmic areas, suggesting that Cas9/gRNA has also caused InDel mutations in the input HSV-1 DNA, thus eradicating expression of ICPO from the incoming virus.

The impact of these mutations on the ability of ICPO to stimulate HSV-1 replication was assessed by comparing ΔICP0 HSV-1 replication levels in these clones (InDel 2 and InDel 3) to those seen in control cells expressing the wild-type proteins. Supernatants of each infected cell culture were collected 48 hours after infecting cells with ΔICP0 HSV-1, and virus titer was determined by plaque assay. Clones InDel 2 and InDel 3 showed marked declines in ability to support replication of ΔICP0 HSV-1 (58.9% and 80%, respectively), indicating Cas9/gRNA 2A-mediated functional inactivation of ICPO in L7-cells (Figure 7G). Production of gRNA and Cas9 was verified by RT-PCR and Western blot analysis, respectively (Figure 12).

### Impact of Cas9/gRNA on PML nuclear body structure

Promyelocytic leukemia (PML) nuclear bodies are punctate structures localized within nuclei of many eukaryotic cells that control cell growth and transformation (Sahin et al., 2014, J Pathol, 234: 289-291), and also inhibit infection of cells by many viruses including HSV-1 (Wang et al., 2012, Protein Cell, 3: 372-382). Interaction of ICPO with HSV-1 with PML via its N-terminus apparently suppresses PML-mediated anti-viral activity, by disintegrating PML structure (Cuchet-Lourenco et al., 2012, J Virol, 86: 11209-11222; Everett et al, 2006, J Virol, 80: 7995-8005).

PML-associated nuclear bodies punctate structures were evident by fluorescent microscopy in the nuclei of HSV-1-uninfected L7 cells expressing only Cas9 (Figure 8), but infection of these cells with HSV-1/GFP completely changed the structure of PML punctate structures coincident with HSV-1 localization. In contrast, co-expression of both Cas9 and gRNAs in infected L7 cells restored the pattern of PML-like punctate structures to one very similar to that seen in uninfected cells. Low levels of GFP were also detected in the cytoplasm, probably representing expression of GFP encoded by the viral genome in the absence of its productive infection cycle (Figure 8). This observation suggests that Cas9/gRNA-induced mutation of ICPO interferes with its PML body-disrupting antiviral activity.

### Expression of Cas9/gRNA suppresses HSV-1 infection of human cell line

Seeking a convenient human cell line in which to further explore the anti-HSV-1 activity of the present CRISPR/Cas9 system, it was found that the human oligodendroglioma cell line TC620 (Merrill and Matsushima, 1988, J Biol Regul Homeost Agents, 2: 77-86) robustly supported HSV-1 replication at low and high multiplicities of infection, as evidenced by expression of ICPO, the early viral protein ICP8, which is involved in viral DNA replication and late gene transactivation (Challberg 1986, Proc Natl Acad Sci USA, 83: 9094-9098; Gao and Knipe, 1991, J Virol 65, 2666-2675; Tanguy Le Gac et al., 1998, J Biol Chem, 273: 13801-13807), and the late envelope protein, glycoprotein C, which enhances infectivity of the virus (Friedman et al. 1984, Nature, 309: 633-635) (Figure 13A - Figure 13D).

Several clonal TC620 cell sublines that stably expressed Cas9 or Cas9 plus gRNA 2A were created. These cell sublines were used to test the ability of Cas9/gRNA 2A to suppress HSV-1 infection. The clonal lines 6 and 10, where both Cas9 and gRNAs are produced, showed a substantial decrease in the level of ICPO production, seen in Western blots, whereas cells that only express Cas9 did not show any effect in the ICPO production level (Figure 9A). It was confirmed using fluorescence microscopy for HSV-1/GFP that Cas9/gRNA 2A expression suppressed HSV-1 infection of TC620 cells. Supernatants collected after HSV-1 infection from control cells expressing only Cas9, and from clones 6 and 10, were compared using plaque assay with Vero cells at 1/10 and 1/100 dilutions (Figure 9C). It was found that Cas9/gRNA 2A suppressed plaque formation in clones 6 and 10, respectively, by 85% and 72% at 1/10 dilution, and by 95% and 90% at 1/100 dilution (Figure 9C and Figure 9D). The enhanced suppression at the higher dilution concords with prior findings that the inhibitory effects of ICPO are more pronounced at lower-MOI infection (Chen and Silverstein, 1992, J Virol, 66: 2916-2927). These data indicate that in uninfected cells, Cas9/gRNA 2A protects cells against HSV-1 infection, by inhibiting ICPO production and hence its suppression of host antiviral defenses, contributing to lytic infection.

The potential effects of Cas9/gRNA 2A on several indices of cellular health status were also assessed. Expression of Cas9, either alone or together with gRNA 2A, had no significant effect on cell cycle progression of TC620 cells (Figure 14A). Similarly, immunofluorescent staining of cells with annexin indicated no major impacts of Cas9 or Cas9/gRNA 2A on apoptosis (Figure 14B). Accordingly, no adverse effects of the gene-editing system on cell viability were observed (Figure 14C). To evaluate the specificity of gRNA 2A and determine whether the present gene editing strategy induced any off-target effects or compromises host genome integrity, the SURVEYOR assay was used. The results showed no evidence for InDel mutations in any potential exonic off-target sites in several representative human genes that were identified by bioinformatic screening using a shorter (13 nt seed sequence corresponding to the 2A sequence of HSV-1 (Figure 15, also see Table 4). Further, results from gene amplification followed by DNA sequencing of the potential cellular off-targets genes, as noted above, confirmed no off-target effects of the HSV-1 aimed gene editing system (Figure 15B).

### Lentivirus-mediated delivery of Cas9 and gRNA suppresses HSV- 1 infection and protects cells from infection

To further improve delivery efficiency and evaluate anti-HSV-1 activity of the described gene editing molecules, TC620 cells expressing Cas9 were infected with HSV-1 and 36 hours later, HSV-1 infected cells were transduced with lentivirus (LV) expressing gRNA 2A or 2B in single or duplex configuration. Results showed markedly suppressed expression of ICPO, ICP8, and the late glycoprotein C (Figure 10A), indicating that the incoming gRNAs by lentivirus suppressed HSV-1 protein production in Cas9-expressing cells. Treating the cells with LVgRNA 2A and LVgRNA 2B, drastically suppressed viral production of infected cells (by 97% and 82%, respectively), as measured by plaque assay of infectious virus in culture supernatants (Figure 10B and Figure 10C). Throughout these studies, it was noticed that gRNA 2A is slightly more effective than gRNA 2B in editing the ICPO gene and suppressing HSV-1 replication. To assess the ability of Cas9 and gRNAs 2A, 2B or both in protecting cells from HSV-1 infection, normal TC260 cells were transduced with lentivirus expressing Cas9 (LVCas9), along with lentivirus expressing gRNAs (LVgRNAs) 2A, 2B, or both. It was found that LVCas9/LVgRNA 2A significantly decreased viral protein expression (Figure 10D) confirmed by the results from fluorescence microscopy showing great suppression of GFP stained cells (Figure 10E) and a drastic decline in viral abundance as evaluated by plaque assay (Figure 10F). ICP4 and ICP27 are the two other immediate early (IE) regulatory proteins that are largely responsible for the transition from the IE to the early phase of viral gene transcription, and regulatory viral and cellular mRNA processing events and activation of late genes, respectively (Knipe et al., 2008, Nature Rev., 6: 211-221). In the next series of experiments, after bioinformatic screening of the ICP4 and ICP27 genes, gRNAs with no prediction of off-target effects were selected and their ability to collaboratively suppress HSV-1 infection of TC260 cells was assessed. As shown in Figure 16, lentivirus-mediated delivery of ICPO gRNA 2A and ICP4 gRNA m1, or ICPO gRNA 2A and ICP27 gRNA m1 completely abolished HSV-1 infection of TC260 cells. Interestingly, residual infected cells, which is frequently seen in ICPO gRNA treated cells, were not detected in the combination treatment of these cells (compare Figure 16D and Figure 16E to Figure 16C).

**Table 4: Predicted ICPO gRNAs and their off-target numbers (100% match)**

| | gRNA sequence | **20** | **19** | **18** | **17** | **16** | **15** | **14** | **13** | **12** |
|---|---|---|---|---|---|---|---|---|---|---|
| gRNA 2A | 1. TCCCTGCGACCGAGACCTGCCGG (SEQ ID NO: 34) | 0 | 0 | 0 | 0 | 0 | 0 | 0 | 7 | 19 |
| gRNA 2B | 2. ACACGGAACTGTTCGAGACGGGG (SEQ ID NO: 35) | 0 | | 0 | 0 | 0 | 0 | 0 | 0 | 0 |
| gRNA 2C | 3.GCATCCCGTGCATGAAAACCTGG (SEQ ID NO; 36) | 0 | 0 | 0 | 0 | 0 | 0 | 0 | 8 | 31 |
| The genomic sequence of HSV-1 ICPO (NC_ 0018061) was used to search for Cas9/gRNA target sites containing a 20-bp guide sequence plus the protospacer adjacent motif sequence (NGG) using a CRISPR/Cas9 finder tool | | | | | | | | | | |
| NGG (AGG, TGG, GGG, CGG) was blasted against available human genomic and transcript sequence with 1000 aligned sequences being displayed. The Target sequences (1-23 through 9-23 nucleotides) were searched to find the number of genomic targets with 100% match. The number of off-targets for each searching was divided by 3 because of repeated genome library. The number shown indicates the sum of four search (NGG). The bold number indicates the gRNAs target sequences farthest from NGG | | | | | | | | | | |

### CRISPR/Cas9 system targeting ICPO to permanently suppress HSV-1 infection

Current therapy to suppress HSV-1 replication includes nucleoside analogues that target the viral thymidine kinase (TK), including acyclovir and its derivatives. Such agents help control the symptoms of primary HSV-1 infection, but do not eliminate latent HSV-1 infection. Further, development of HSV-1 strains that are resistant to TK inhibitors, due to mutations in the TK genes, remains a clinical challenge in the treatment of HSV-1 associated illness.

The experiments presented herein tested the ability of CRISPR/Cas9 system to permanently suppress HSV-1 infection by altering the viral genes responsible for the immediate early phage of the infection cycle. ICPO was chosen as the target for editing as it controls the balance between HSV-1 latency and replication and plays an important role in blocking several cell-mediated anti-viral activities including PML nuclear bodies (Chee et al., 2003, J Virol 77: 7101-7105). Moreover, ICPO of HSV-1 is highly sensitive to inhibition by interferon α and β, and elicits strong immune responses against wild-type HSV-1 (Halford et al, 2006, Virol J, 3: 44). Thus, abrogating ICPO expression can indirectly interfere with replication of new HSV-1 infection. The present findings indicate that targeting the ICPO gene using CRISPR/Cas9, induced multiple InDel mutations in exon II of ICPO, halted disruption of the endogenous antiviral PML assemblies, and completely abrogates the viral life cycle in the infected cells.

Among the three gRNAs designed and presented herein for ICPO gene targeting by CRISPR/Cas9, gRNA 2A showed the greatest potency in single configuration to compromise the structure of exon II of the ICPO genome by inducing InDel mutations that led to the generation of frameshift in the ICPO open reading frame. This mutation in exon II also interfered with ICPO interaction with PML and formation of nuclear bodies. Thus, Cas9/gRNA 2A exerts its antiviral activity by inhibiting expression of multiple HSV-1 genes whose products are required for the infection cycle and halting of host-derived anti-viral events including the assembly of PML bodies. Lentivirus-mediated delivery of Cas9 and gRNAs during viral infection significantly decreased viral loads produced by infected cells. Results from combinatory experiments presented herein using ICPO gRNA plus either ICP4 gRNA or ICP27 gRNA showed complete elimination of HSV1 replication in the infected cells. This observation suggests that elimination of HSV-1 at the latent and productive stages may require editing of multiple viral genes, an approach that is achievable considering the simplicity and flexibility of the CRISPR/Cas9 strategy. The combined strategy also mitigates concern related to repair of one hit or possible recombination, should an HSV-based delivery system be used. Further, it was found that Cas9 expression in cells prior to infection suppressed HSV-1 replication when gRNA was introduced to the cells by lentivirus, suggesting that the Cas9 strategy is applicable to protecting cells against HSV infection. In this respect, one can envision development of a novel therapeutic strategy in which Cas9 gene only becomes activated by a HSV-1 immediate early protein such as ICP4, thus allowing Cas9 to remain silent in the absence of HSV-1 and reactivated only when the virus begins to enter the lytic phase of infection.

The prospects for translation of Cas9/CRISPR-based viral targeting to human clinical therapies depend not only upon development of efficient delivery systems, but also upon their specificity. As a first step to assessing this critical issue in the HSV-targeting system in vitro, its effects were surveyed on key cell functions, indices of cell health, and potential genomic targets. It was found that the Cas9/gRNA system lacks adverse effects upon cell viability and processes including the cell cycle, does not induce apoptosis, and does not jeopardize cell viability. To achieve this level of safety, avoid effects on human translated genomic sites and exclude any transcription DNA motif, bioinformatic screening was employed based on the strictest 12 bp plus PAM target selection criteria. The most effective gRNA (2A) was 30 nt in length plus NGG. It showed no evidence for InDel mutation in any of a series of five representative off-target host genes identified by bioinformatic screening using shorter (12 nt) seed sequences corresponding to target A of ICPO exon II.

The present results also show that the CRISPR/Cas9 system can be refined and used for protecting cells against HSV-1 infection. The pre-existence of Cas9 plus gRNAs 2A in human cells that robustly support HSV-1 replication prevented efficient replication of the incoming HSV-1. This observation is reminiscent of the strategy by which prokaryotic cells protect themselves from foreign DNA elements such as transposable elements and bacteriophages (Horvath and Barrangou, 2010, Science, 327: 167-170). Given the ease and rapidity of the CRISPR/Cas9 gene editing system and the complexity of HSV-1 lytic infection cycle, a combination therapy can be developed that includes a cocktail of gRNAs for targeting important viral proteins involved in the regulation of the immediate early, early and late phases of HSV-1 infection. For delivering Cas9/gRNAs, there are several virus-based systems available including lentivirus, AAV, modified HSV-1 vectors. Non-viral delivery systems including nanoparticles and extracellular vesicles may also be used. In addition, expression of Cas9 can be modulated through various inducible expression systems to control its expression and expedite Cas9 turnover, which helps to limit off-target effects.

The data presented herein demonstrate that the present Cas9/gRNA editing system targeting ICPO introduced mutations in this immediate early gene of HSV-1 with high specificity, suspends the HSV-1 lytic infection cycle, and protects uninfected cells from HSV-1 infection. The results show that introducing a single nucleotide mismatch in the ICPO coding sequence by this gene editing strategy is highly effective in blocking viral replication. Thus, these studies demonstrate that CRISPR/Cas9 can be developed as a new therapeutic tool for HSV-1 infection and a potential and permanent strategy for eliminating HSV-1 DNA from latently infected cells.

## Claims

1. A composition for use in treating or preventing a herpesvirus infection comprising:
(a) an isolated nucleic acid encoding a CRISPR-associated (Cas) peptide; and
(b) one or more isolated nucleic acids, wherein the one or more isolated nucleic acids encode multiple guide nucleic acids, wherein each guide nucleic acid comprises a nucleotide sequence complementary to a different target sequence in the herpesvirus genome, and wherein a guide nucleic acid of the multiple guide nucleic acids comprises a target sequence complementary to a sequence within the ICPO domain of the herpesvirus genome and wherein a second guide nucleic acid of the multiple guide nucleic acids comprises a target sequence complementary to a sequence within the ICP4 or ICP27 domain of the herpesvirus genome.

2. The composition for use according to claim 1, wherein the Cas peptide is Cas9 or a variant thereof.

3. The composition for use according to claim 2, wherein the Cas9 variant comprises one or more point mutations, relative to wildtype Streptococcus pyogenes Cas9 (spCas9), selected from the group consisting of: R780A, K810A, K848A, K855A, H982A, K1003A, R1060A, D1 135E, N497A, R661A, Q695A, Q926A, L169A, Y450A, M495A, M694A, and M698A.

4. The composition for use according to claim 1, wherein the Cas peptide is Cpfl or a variant thereof.

5. The composition for use according to any one of claims 1-4, wherein isolated nucleic acid encoding the Cas peptide is optimized for expression in a human cell.

6. The composition for use according to any one of claims 1-5, wherein the guide nucleic acid is RNA.

7. The composition for use according to any one of claims 1-6, wherein the guide nucleic acid comprises crRNA and tracrRNA.

8. The composition for use according to any one of claims 1-7, wherein the herpesvirus is selected from the group consisting of herpes simplex type I (HSV1), herpes simplex virus 2 (HSV2), human herpresvirus-3 (HHV-3; varicella zoster virus (VZV), human herpesvirus-4 (HHV-4; Epstein-Barr virus (EBV)), human herpesvirus-5 (HHV-5; Cytomegalovirus (CMV)), human herpesvirus-6 (HHV-6; roseolovirus), human herpes virus-7 (HHV-7), and human herpesvirus-8 (HHV-8; Karposi's sarcoma-associated herpesvirus (KSHV)).

9. The composition for use according to any one of claims 1-8, wherein the target sequence is selected from the group consisting of SEQ ID NO: 1, SEQ ID NO: 2, SEQ ID NO: 3, SEQ ID NO: 4, SEQ ID NO: 5, and SEQ ID NO: 6.

10. A pharmaceutical composition comprising:
(a) an isolated nucleic acid encoding a CRISPR-associated (Cas) peptide; and
(b) one or more isolated nucleic acids, wherein the one or more isolated nucleic acids encode multiple guide nucleic acids, wherein each guide nucleic acid comprises a nucleotide sequence complementary to a different target sequence in the herpesvirus genome, and wherein a guide nucleic acid of the multiple guide nucleic acids comprises a target sequence complementary to a sequence within the ICPO domain of the herpesvirus genome and wherein a second guide nucleic acid of the multiple guide nucleic acids comprises a second target sequence complementary to a sequence within the ICP4 or ICP27 domain of the herpesvirus genome.

11. An expression vector encoding a CRISPR-associated (Cas) peptide and one or more isolated nucleic acids, wherein the one or more isolated nucleic acids encode multiple guide nucleic acids, wherein each guide nucleic acid comprises a nucleotide sequence complementary to a different target sequence in the herpesvirus genome, and wherein a guide nucleic acid of the multiple guide nucleic acids comprises a target sequence complementary to a sequence within the ICPO domain of the herpesvirus genome and wherein a second guide nucleic acid of the multiple guide nucleic acids comprises a second target sequence complementary to a sequence within the ICP4 or ICP27 domain of the herpesvirus genome.

12. A host cell comprising the expression vector of claim 11.

## Patentansprüche

1. Zusammensetzung zur Verwendung beim Behandeln oder Verhindern einer Herpesvirusinfektion, umfassend:
(a) eine ein CRISPR-assoziiertes (Cas-) Peptid codierende isolierte Nukleinsäure; und
(b) mindestens eine isolierte Nukleinsäure, wobei die mindestens eine isolierte Nukleinsäure mehrere Leitnukleinsäuren codiert, wobei jede Leitnukleinsäure eine Nukleotidsequenz umfasst, die zu einer anderen Zielsequenz im Herpesvirusgenom komplementär ist, und wobei eine Leitnukleinsäure der mehreren Leitnukleinsäuren eine Zielsequenz umfasst, die zu einer Sequenz in der ICPO-Domäne des Herpesvirusgenoms komplementär ist, und wobei eine zweite Leitnukleinsäure der mehreren Leitnukleinsäuren eine Zielsequenz umfasst, die zu einer Sequenz in der ICP4- oder ICP27-Domäne des Herpesvirusgenoms komplementär ist.

2. Zusammensetzung zur Verwendung nach Anspruch 1, wobei es sich bei dem Cas-Peptid um Cas9 oder eine Variante davon handelt.

3. Zusammensetzung zur Verwendung nach Anspruch 2, wobei die Cas9-Variante mindestens eine Punktmutation relativ zum Wildtyp-Cas9 von Streptococcus pyogenes (spCas9) umfasst, ausgewählt aus der Gruppe bestehend aus: R780A, K810A, K848A, K855A, H982A, K1003A, R1060A, D1 135E, N497A, R661A, Q695A, Q926A, L169A, Y450A, M495A, M694A und M698A.

4. Zusammensetzung zur Verwendung nach Anspruch 1, wobei es sich bei dem Cas-Peptid um Cpfl oder eine Variante davon handelt.

5. Zusammensetzung zur Verwendung nach einem der Ansprüche 1 bis 4, wobei eine das Cas-Peptid codierende isolierte Nukleinsäure für die Expression in einer menschlichen Zelle optimiert ist.

6. Zusammensetzung zur Verwendung nach einem der Ansprüche 1 bis 5, wobei es sich bei der Leitnukleinsäure um RNA handelt.

7. Zusammensetzung zur Verwendung nach einem der Ansprüche 1 bis 6, wobei die Leitnukleinsäure crRNA und tracrRNA umfasst.

8. Zusammensetzung zur Verwendung nach einem der Ansprüche 1 bis 7, wobei das Herpesvirus aus der Gruppe bestehend aus Herpes simplex Typ I (HSV1), Herpes-simplex-Virus 2 (HSV2), humanem Herpesvirus 3 (HHV-3; Varizella-Zoster-Virus (VZV)), humanem Herpesvirus 4 (HHV-4; Epstein-Barr-Virus (EBV)), humanem Herpesvirus 5 (HHV-5; Cytomegalovirus (CMV)), humanem Herpesvirus 6 (HHV-6; Roseolovirus), humanem Herpesvirus 7 (HHV-7) und humanem Herpesvirus 8 (HHV-8; Karposi-Sarkomassoziiertes Herpesvirus (KSHV)) ausgewählt ist.

9. Zusammensetzung zur Verwendung nach einem der Ansprüche 1 bis 8, wobei die Zielsequenz aus der Gruppe bestehend aus SEQ ID NO: 1, SEQ ID NO: 2, SEQ ID NO: 3, SEQ ID NO: 4, SEQ ID NO: 5 und SEQ ID NO: 6 ausgewählt ist.

10. Pharmazeutische Zusammensetzung, umfassend:
(a) eine ein CRISPR-assoziiertes (Cas-) Peptid codierende isolierte Nukleinsäure; und
(b) mindestens eine isolierte Nukleinsäure, wobei die mindestens eine isolierte Nukleinsäure mehrere Leitnukleinsäuren codiert, wobei jede Leitnukleinsäure eine Nukleotidsequenz umfasst, die zu einer anderen Zielsequenz im Herpesvirusgenom komplementär ist, und wobei eine Leitnukleinsäure der mehreren Leitnukleinsäuren eine Zielsequenz umfasst, die zu einer Sequenz in der ICPO-Domäne des Herpesvirusgenoms komplementär ist, und wobei eine zweite Leitnukleinsäure der mehreren Leitnukleinsäuren eine zweite Zielsequenz umfasst, die zu einer Sequenz in der ICP4- oder ICP27-Domäne des Herpesvirusgenoms komplementär ist.

11. Expressionsvektor codierend ein CRISPR-assoziiertes (Cas-) Peptid und mindestens eine isolierte Nukleinsäure, wobei die mindestens eine isolierte Nukleinsäure mehrere Leitnukleinsäuren codiert, wobei jede Leitnukleinsäure eine Nukleotidsequenz umfasst, die zu einer anderen Zielsequenz im Herpesvirusgenom komplementär ist, und wobei eine Leitnukleinsäure der mehreren Leitnukleinsäuren eine Zielsequenz umfasst, die zu einer Sequenz in der ICPO-Domäne des Herpesvirusgenoms komplementär ist, und wobei eine zweite Leitnukleinsäure der mehreren Leitnukleinsäuren eine zweite Zielsequenz umfasst, die zu einer Sequenz in der ICP4- oder ICP27-Domäne des Herpesvirusgenoms komplementär ist.

12. Wirtszelle umfassend den Expressionsvektor nach Anspruch 11.

## Revendications

1. Composition destinée à être utilisée dans le traitement ou la prévention d'une infection par virus de l'herpès comprenant :
(a) un acide nucléique isolé codant pour un peptide (Cas) associé à CRISPR ; et
(b) un ou plusieurs acides nucléiques isolés, les un ou plusieurs acides nucléiques isolés codant pour des acides nucléiques guides multiples, chaque acide nucléique guide comprenant une séquence nucléotidique complémentaire à une séquence cible différente dans le génome du virus de l'herpès et un acide nucléique guide des acides nucléiques guides multiples comprenant une séquence cible complémentaire à une séquence dans le domaine ICPO du génome du virus de l'herpès et un second acide nucléique guide des acides nucléiques guides multiples comprenant une séquence cible complémentaire à une séquence dans le domaine ICP4 ou ICP27 du génome du virus de l'herpès.

2. Composition destinée à être utilisée selon la revendication 1, dans laquelle le peptide Cas est Cas9 ou un de ses variants.

3. Composition destinée à être utilisée selon la revendication 2, dans laquelle le variant de Cas9 comprend une ou plusieurs mutations ponctuelles, par rapport au Cas9 de Streptococcus pyogenes sauvage (spCas9) choisi dans le groupe constitué par : R780A, K810A, K848A, K855A, H982A, K1003A, R1060A, D1 135E, N497A, R661A, Q695A, Q926A, L169A, Y450A, M495A, M694A et M698A.

4. Composition destinée à être utilisée selon la revendication 1, dans laquelle le peptide Cas est Cpfl ou un de ses variants.

5. Composition destinée à être utilisée selon l'une quelconque des revendications 1 à 4, dans laquelle l'acide nucléique isolé codant pour le peptide Cas est optimisé pour l'expression dans une cellule humaine.

6. Composition destinée à être utilisée selon l'une quelconque des revendications 1 à 5, dans laquelle l'acide nucléique guide est un ARN.

7. Composition destinée à être utilisée selon l'une quelconque des revendications 1 à 6, dans laquelle l'acide nucléique guide comprend un ARNcr et un ARNtracr.

8. Composition destinée à être utilisée selon l'une quelconque des revendications 1 à 7, dans laquelle le virus de l'herpès est choisi dans le groupe constitué par le type I d'herpès simplex (HSV1), le virus herpès simplex 2 (HSV2), le virus de l'herpès 3 humain (HHV-3 ; le virus varicella zoster (VZV)), le virus de l'herpès 4 humain (HHV-4 ; le virus d'Epstein-Barr (EBV)), le virus de l'herpès 5 humain (HHV-5 ; le cytomégalovirus (CMV)), le virus de l'herpès 6 humain (HHV-6 ; roséolovirus) le virus de l'herpès 7 humain (HHV-7) et le virus de l'herpès 8 humain (HHV-8 ; virus de l'herpès associé au sarcome de Kaposi (KSHV)).

9. Composition destinée à être utilisée selon l'une quelconque des revendications 1 à 8, dans laquelle la séquence cible est choisie dans le groupe constitué par SEQ ID NO: 1, SEQ ID NO: 2, SEQ ID NO: 3, SEQ ID NO: 4, SEQ ID NO: 5 et SEQ ID NO: 6.

10. Composition pharmaceutique comprenant :
(a) un acide nucléique isolé codant pour un peptide (Cas) associé à CRISPR ; et
(b) un ou plusieurs acides nucléiques isolés, les un ou plusieurs acides nucléiques isolés codant pour des acides nucléiques guides multiples, chaque acide nucléique guide comprenant une séquence nucléotidique complémentaire à une séquence cible différente dans le génome du virus de l'herpès et un acide nucléique guide des acides nucléiques guides multiples comprenant une séquence cible complémentaire à une séquence dans le domaine ICPO du génome du virus de l'herpès et un second acide nucléique guide des acides nucléiques guides multiples comprenant une seconde séquence cible complémentaire à une séquence dans le domaine ICP4 ou ICP27 du génome du virus de l'herpès.

11. Vecteur d'expression codant pour un peptide (Cas) associé à CRISPR et un ou plusieurs acides nucléiques isolés, les un ou plusieurs acides nucléiques isolés codant pour des acides nucléiques guides multiples, chaque acide nucléique guide comprenant une séquence nucléotidique complémentaire à une séquence cible différente dans le génome du virus de l'herpès et un acide nucléique guide des acides nucléiques guides multiples comprenant une séquence cible complémentaire à une séquence dans le domaine ICPO du génome du virus de l'herpès et un second acide nucléique guide des acides nucléiques guides multiples comprenant une seconde séquence cible complémentaire à une séquence dans le domaine ICP4 ou ICP27 du génome du virus de l'herpès.

12. Cellule hôte comprenant le vecteur d'expression selon la revendication 11.
